# FASCICULE DE BREVET EUROPEEN

(11) **EP 1 383 890 B1**
(45) Date de publication et mention de la délivrance du brevet: **14.05.2008**
(21) Numéro de dépôt: 02761933.7
(22) Date de dépôt: 10.04.2002
(51) Int. Cl.: C12N 15/10, C12N 15/01, C12N 15/54, C12N 15/70, C12N 9/12, C12N 1/21

(54) **MUTANTS DE LA DESOXYCYTIDINE KINASE POSSEDANT UNE ACTIVITE ENZYMATIQUE ELARGIE**
DESOXYCYTIDINKINASE-MUTANTEN MIT ERWEITERTER ENZYMAKTIVITÄT
MUTANTS OF DESOXYCYTIDINE KINASE HAVING EXTENDED ENZYMATIC ACTIVITY

(30) Priorité: 10.04.2001 FR 0104856
(43) Date de publication de la demande: 28.01.2004
(60) Demande divisionnaire: 07076009.5
(73) Titulaire: INSTITUT PASTEUR, 75015 Paris (FR); CENTRE NATIONAL DE LA RECHERCHE SCIENTIFIQUE (CNRS), 75016 Paris (FR)
(72) Inventeur: MARLIERE, Philippe, F-75012 Paris (FR); POCHET, Sylvie, 75012 Paris (FR); BOUZON, Madeleine, 92190 Meudon (FR)
(74) Mandataire: Santarelli
(86) Numéro de dépôt international: PCT/FR2002/001252
(87) Numéro de publication internationale: WO 2002/083892

(56) Documents cités:
- EP-A- 0 939 130
- WO-A1-95/22622
- WO-A1-97/29196
- WO-A2-01/88106
- WANG JIANGHAI ET AL: "An Escherichia coli system expressing human deoxyribonucleoside salvage enzymes for evaluation of potential antiproliferative nucleoside analogs." ANTIMICROBIAL AGENTS AND CHEMOTHERAPY, vol. 42, no. 10, octobre 1998 (1998-10), pages 2620-2625, XP002193076 ISSN: 0066-4804
- BOUZON M ET AL: "Human deoxycytidine kinase as a conditional mutator in Escherichia coli" COMPTES RENDUS DES SEANCES DE L'ACADEMIE DES SCIENCES. SERIE III: SCIENCES DE LA VIE, ELSEVIER, AMSTERDAM, NL, vol. 320, no. 6, juin 1997 (1997-06), pages 427-434, XP004270078 ISSN: 0764-4469 cité dans la demande
- TOKUNAGA TETSUHIRO ET AL: "Efficacy of trimethoprim in murine experimental infection with a thymidine kinase-deficient mutant of Escherichia coli." ANTIMICROBIAL AGENTS AND CHEMOTHERAPY, vol. 41, no. 5, 1997, pages 1042-1045, XP002193077 ISSN: 0066-4804
- WANG JIANGHAI ET AL: "Expression of human mitochondrial thymidine kinase in Escherichia coli: Correlation between the enzymatic activity of pyrimidine nucleoside analogues and their inhibitory effect on bacterial growth." BIOCHEMICAL PHARMACOLOGY, vol. 59, no. 12, 15 juin 2000 (2000-06-15), pages 1583-1588, XP002193078 ISSN: 0006-2952
- CHOTTINER E G ET AL: "CLONING AND EXPRESSION OF HUMAN DEOXYCYTIDINE KINASE CDNA" PROCEEDINGS OF THE NATIONAL ACADEMY OF SCIENCES OF USA, NATIONAL ACADEMY OF SCIENCE. WASHINGTON, US, vol. 88, février 1991 (1991-02), pages 1531-1535, XP000941361 ISSN: 0027-8424
- OKAJIMA TOSHIHIDE ET AL: "Site-directed mutagenesis of AMP-binding residues in adenylate kinase: Alteration of substrate specificity." FEBS (FEDERATION OF EUROPEAN BIOCHEMICAL SOCIETIES) LETTERS, vol. 334, no. 1, 1993, pages 86-88, XP002193079 ISSN: 0014-5793 cité dans la demande
- LIANGLU WAN ET AL: 'In vitro evolution of horse heart myoglobin to increase peroxidase activity' PROC. NATL. ACAD SCI. vol. 95, Octobre 1998, US, pages 12825 - 12831, XP002297538
- SOUSA R.; PADILA R.: 'A mutant T7 RNA polymerase as a DNA polymerase' THE EMBO JOURNAL vol. 14, no. 18, 1995, OXFORD UNIVERSITY PRESS, UK, pages 4609 - 4621
- KOSTYUK D.A. ET AL: 'Mutants of T7 RNA polymerase that are able to synthesize both RNA and DNA' FEBS LETTERS vol. 369, 1995, ELSEVIER, AMSTERDAM, NL, pages 165 - 168

### LISTE DE SEQUENCES

<110> Institut Pasteur
   Centre National de la Recherche Scientifique CNRS
<120> Mutants de la desoxycitidine kinase possédant une activité enzymatique élargie
<130> D18773
<140> FR 01 04856
   <141> 2001-04-10
<160> 3
<170> PatentIn Ver. 2.1
<210> 1
   <211> 260
   <212> PRT
   <213> Homo sapiens
<220>
   <223> DCK1 humaine D133E
<400> 1
   Met Ala Thr Pro Pro Lys Arg Ser Cys Pro Ser Phe Ser Ala Ser Ser
    1               5                  10                  15
   Glu Gly Thr Arg Ile Lys Lys Ile Ser Ile Glu Gly Asn Ile Ala Ala
               20                  25                  30
   Gly Lys Ser Thr Phe Val Asn Ile Leu Lys Gln Leu Cys Glu Asp Trp
           35                  40                  45
   Glu Val Val Pro Glu Pro Val Ala Arg Trp Cys Asn Val Gln Ser Thr
       50                  55                  60
   Gln Asp Glu Phe Glu Glu Leu Thr Met Ser Gln Lys Asn Gly Gly Asn
   65                  70                  75                  80
   Val Leu Gln Met Met Tyr Glu Lys Pro Glu Arg Trp Ser Phe Thr Phe
                   85                  90                  95
   Gln Thr Tyr Ala Cys Leu Ser Arg Ile Arg Ala Gln Leu Ala Ser Leu
               100                 105                 110
   Asn Gly Lys Leu Lys Asp Ala Glu Lys Pro Val Leu Phe Phe Glu Arg
           115                 120                 125
   Ser Val Tyr Ser Glu Arg Tyr Ile Phe Ala Ser Asn Leu Tyr Glu Ser
       130                 135                 140
   Glu Cys Met Asn Glu Thr Glu Trp Thr Ile Tyr Gln Asp Trp His Asp
   145                 150                 155                 160
   Trp Met Asn Asn Gln Phe Gly Gln Ser Leu Glu Leu Asp Gly Ile Ile
                   165                 170                 175
   Tyr Leu Gln Ala Thr Pro Glu Thr Cys Leu His Arg Ile Tyr Leu Arg
               180                 185                 190
   Gly Arg Asn Glu Glu Gln Gly Ile Pro Leu Glu Tyr Leu Glu Lys Leu
           195                 200                 205
   His Tyr Lys His Glu Ser Trp Leu Leu His Arg Thr Leu Lys Thr Asn
       210                 215                 220
   Phe Asp Tyr Leu Gln Glu Val Pro Ile Leu Thr Leu Asp Val Asn Glu
   225                 230                 235                 240
   Asp Phe Lys Asp Lys Tyr Glu Ser Leu Val Glu Lys Val Lys Glu Phe
                   245                 250                 255
   Leu Ser Thr Leu
               260
<210> 2
   <211> 260
   <212> PRT
   <213> Homo sapiens
<220>
   <223> DCK1 humaine R104Q
<400> 2
   Met Ala Thr Pro Pro Lys Arg Ser Cys Pro Ser Phe Ser Ala Ser Ser
   1               5                   10                  15
   Glu Gly Thr Arg Ile Lys Lys Ile Ser Ile Glu Gly Asn Ile Ala Ala
               20                  25                  30
   Gly Lys Ser Thr Phe Val Asn Ile Leu Lys Gln Leu Cys Glu Asp Trp
           35                  40                  45
   Glu Val Val Pro Glu Pro Val Ala Arg Trp Cys Asn Val Gln Ser Thr
       50                  55                  60
   Gln Asp Glu Phe Glu Glu Leu Thr Met Ser Gln Lys Asn Gly Gly Asn
   65                  70                  75                  80
   Val Leu Gln Met Met Tyr Glu Lys Pro Glu Arg Trp Ser Phe Thr Phe
                   85                  90                  95
   Gln Thr Tyr Ala Cys Leu Ser Gln Ile Arg Ala Gln Leu Ala Ser Leu
               100                 105                 110
   Asn Gly Lys Leu Lys Asp Ala Glu Lys Pro Val Leu Phe Phe Glu Arg
           115                 120                 125
   Ser Val Tyr Ser Asp Arg Tyr Ile Phe Ala Ser Asn Leu Tyr Glu Ser
       130                 135                 140
   Glu Cys Met Asn Glu Thr Glu Trp Thr Ile Tyr Gln Asp Trp His Asp
   145                 150                 155                 160
   Trp Met Asn Asn Gln Phe Gly Gln Ser Leu Glu Leu Asp Gly Ile Ile
                   165                 170                 175
   Tyr Leu Gln Ala Thr Pro Glu Thr Cys Leu His Arg Ile Tyr Leu Arg
                       180                 185                 190
   Gly Arg Asn Glu Glu Gln Gly Ile Pro Leu Glu Tyr Leu Glu Lys Leu
           195                 200                 205
   His Tyr Lys His Glu Ser Trp Leu Leu His Arg Thr Leu Lys Thr Asn
       210                 215                 220
   Phe Asp Tyr Leu Gln Glu Val Pro Ile Leu Thr Leu Asp Val Asn Glu
   225                 230                 235                 240
   Asp Phe Lys Asp Lys Tyr Glu Ser Leu Val Glu Lys Val Lys Glu Phe
                   245                 250                 255
   Leu Ser Thr Leu
               260
<210> 3
   <211> 260
   <212> PRT
   <213> Homo sapiens
<220>
   <223> DCK1 humaine D133E-R104Q
<400> 3
   Met Ala Thr Pro Pro Lys Arg Ser Cys Pro Ser Phe Ser Ala Ser Ser
   1 5 10 15
   Glu Gly Thr Arg Ile Lys Lys Ile Ser Ile Glu Gly Asn Ile Ala Ala
   20 25 30
   Gly Lys Ser Thr Phe Val Asn Ile Leu Lys Gln Leu Cys Glu Asp Trp
   35 40 45
   Glu Val Val Pro Glu Pro Val Ala Arg Trp Cys Asn Val Gln Ser Thr
   50 55 60
   Gln Asp Glu Phe Glu Glu Leu Thr Met Ser Gln Lys Asn Gly Gly Asn
   65 70 75 80
   Val Leu Gln Met Met Tyr Glu Lys Pro Glu Arg Trp Ser Phe Thr Phe
   85 90 95
   Gln Thr Tyr Ala Cys Leu Ser Gln Ile Arg Ala Gln Leu Ala Ser Leu
   100 105 110
   Asn Gly Lys Leu Lys Asp Ala Glu Lys Pro Val Leu Phe Phe Glu Arg
   115 120 125
   Ser Val Tyr Ser Glu Arg Tyr Ile Phe Ala Ser Asn Leu Tyr Glu Ser
   130 135 140
   Glu Cys Met Asn Glu Thr Glu Trp Thr Ile Tyr Gln Asp Trp His Asp
   145 150 155 160
   Trp Met Asn Asn Gln Phe Gly Gln Ser Leu Glu Leu Asp Gly Ile Ile
   165 170 175
   Tyr Leu Gln Ala Thr Pro Glu Thr Cys Leu His Arg Ile Tyr Leu Arg
   180 185 190
   Gly Arg Asn Glu Glu Gln Gly Ile Pro Leu Glu Tyr Leu Glu Lys Leu
   195 200 205
   His Tyr Lys His Glu Ser Trp Leu Leu His Arg Thr Leu Lys Thr Asn
   210 215 220
   Phe Asp Tyr Leu Gln Glu Val Pro Ile Leu Thr Leu Asp Val Asn Glu
   225 230 235 240
   Asp Phe Lys Asp Lys Tyr Glu Ser Leu Val Glu Lys Val Lys Glu Phe
   245 250 255
   Leu Ser Thr Leu
   260

## Description

La présente invention se rapporte à un procédé d'évolution artificielle *in vivo* de protéines, ledit procédé permettant de faire évoluer une protéine X par complémentation d'une protéine apparentée Y, X et Y appartenant tous deux à la même classe de la nomenclature enzymatique EC ou à des classes voisines. Les mutants D133E et R104Q de la désoxycytidine kinase (DCK) ont été obtenus, chacune de ces mutations conférant l'acquisition de l'activité thymidine kinase par DCK.

Les techniques de séquençage et d'amplification PCR font appel à une gamme de plus en plus diversifiée de nucléosides triphosphates, les monomères activés qui peuvent être condensés par les ADN polymérases. De tels monomères artificiels se distinguent des quatre monomères naturels tant par des altérations chimiques de la base hétérocyclique [Sala et al, 1996] que du sucre pentose et du groupement triphosphate. La préparation des dérivés triphosphate s'effectue généralement à partir des nucléosides correspondants en soumettant l'alcool 5' libre à une phosphorylation puis en condensant le 5' phosphate au pyrophosphate, pour donner le triphosphate. La catalyse de l'étape de phosphorylation par une nucléoside kinase consommant de l'ATP constitue un procédé valide à l'échelle industrielle. Un tel procédé de synthèse n'est envisageable qu'avec des enzymes présentant une activité élargie, c'est à dire des nucléosides kinases capables de phosphoryler n'importe quel nucléoside avec une efficacité similaire. D'une manière plus générale, l'obtention d'enzymes présentant des activités élargies permettrait d'avoir accès à des outils puissants pour toute sorte d'applications biotechnologiques.

Diverses solutions pour effectuer des mutations dirigées dans une molécule d'ADN ont été décrites dans l'état de la technique. Ces techniques consistent à introduire *in vitro* une mutation, une délétion ou une insertion en un site déterminé dans une molécule d'ADN par exemple en utilisant la PCR. Ces diverses techniques sont décrites dans Hall, et al. Protein Eng. 4:601 (1991); Hemsley, et al. Nucleic Acids Research 17:6545-6551 (1989); Ho, et al. Gene 77:51-59 (1989); Hultman, et al. Nucleic Acids Research 18:5107-5112 (1990); Jones, et al. Nature 344:793-794 (1990); Jones, et al. Biotechniques 12:528-533 (1992); Landt, et al. Gene 96:125-128 (1990); Nassal, et al. Nucleic Acids Research 18:3077-3078 (1990); Nelson, et al. Analytical Biochemistry 180:147-151 (1989); Vallette, et al. Nucleic Acids Research 17:723-733 (1989); Watkins, et al, Biotechniques 15:700-704 (1993); Weiner, et al. Gene 126:35-41 (1993). Yao, et al. PCR Methods and Applications 1:205-207 (1992) et dans Weiner et al, Gene 151:1/9-123(1994).

Outre les problèmes techniques rencontrés, il est impossible de savoir à l'avance quelle serait l'effet d'une mutation donnée sur l'activité d'une protéine avec de telles techniques.

D'autres méthodes consistent à introduire des mutations au hasard dans le génome par l'emploi d'agents mutagènes (2-aminopurine, hydroxylamine ou ACRIDINE) et à sélectionner les cellules ou organismes montrant le phénotype recherché. Néanmoins, ces méthodes conduisent à l'introduction de nombreuses mutations, parfois létales, et ne sont pas adaptées à faire évoluer une protéine donnée dans un but précis.

L'art antérieur montre également que l'on peut employer des systèmes *in vivo*, par exemple en utilisant des ADN polymérase exo-, ou d'autres protéines pouvant introduire des mutations (US 6,015,705), mais aucune de ces techniques ne s'apparente à la méthode proposée par la présente invention.

Par ailleurs, différents systèmes d'expression de gènes ont été mis au point pour produire des enzymes humaines telle que la déoxycytidine kinase (DCK1), tels ceux décrits par exemple par Chottiner et al. (1991), PNAS, 88 :1531-1535 ou par Bouzon, M. et al., C.R. Acad Sci. (1997), 320:427-434, ou telle que la thymidine kinase (TK1) Wang et al., 1998, Antimicrob. Agents Chemother., 42(10) :2620-2625 dans des cellules hôtes procaryotes du type *E. coli,* sans modifier leur activité enzymatique.

D'autres enzymes sont décrites comme ayant acquis une activité enzymatique différente par mutagénèse, comme par exemple, celles décrites dans la demande internationale WO 01/88106 (déoxyribosinucléotide kinase), dans Wan-Liaztglu et al. (1998) In vitro evolution of horse heart Myoglobin to increase peroxydase activity, PNAS, 95(22): 12825-831, ou encore dans la demande internationale WO 97/29196 (thymidine kinase). Cependant ces enzymes mutées n'ont pas un spectre d'activité enzymatique élargi.

Plusieurs variants de polymérases obtenus par mutagénèse aléatoire sont décrits comme pouvant avoir un spectre d'activité élargi. C'est notamment le cas dans la demande internationale WO 95/22622, la demande de brevet européen EP 939 130, l'article de Sousa et al. (1995) A Mutant T7 RNA Polymerase as a DNA Polymerase, The EMBO Journal, 14 : 4609-4621, Kostyuk et al. (1995) Mutants of a T7 RNA Polymerase that are able to synthesize both RNA and DNA, FEBS Letters, 369: 165-168.

Pour répondre aux besoins et problèmes évoqués précédemment, la présente invention propose un procédé d'évolution artificielle *in vivo* de protéines. Ce procédé permet de faire évoluer *in vivo* une protéine X par complémentation d'une protéine apparentée Y, X et Y appartenant tous deux à la même classe de la nomenclature enzymatique EC ou à des classes voisines. En effet, on a démontré qu'il est possible de modifier par mutation l'activité d'un enzyme d'une classe, non seulement à l'intérieur d'une même classe, mais également de lui faire acquérir les activités caractérisant les classes voisines (3 premiers chiffres de la nomenclature EC en commun). En d'autres termes, l'invention apporte un procédé pour faire évoluer une protéine X pour qu'elle acquière l'activité d'une autre protéine Y, X conservant au moins une de ses propriétés ou activités initiales et donc présentant *in fine* une activité élargie.

Ce procédé est particulièrement adapté à des enzymes du type nucléotidyl kinase, phosphorylase et nucléotidyl transférases, car on peut mettre à profit leur capacité à introduire des mutations dans leur propre gène.
A ce titre, l'invention a été mise en oeuvre pour la désoxycytidine kinase d'Homo sapiens (DCK) qui est un enzyme capable de phosphoryler une large gamme de nucléosides présentant une parenté chimique avec la désoxycytidine (dC), suivant la réaction :

dC + ATP → dCMP + ADP.

En plus de la désoxycytidine, cet enzyme reconnaît comme substrats les désoxynucléosides puriques dA et dG ainsi que des analogues structuraux de bases ou de sucres, comme la 5-aza-cytidine (5azadC) et l'arabinocytidine. Toutefois, la thymidine n'est pas substrat de l'enzyme *in vitro* [Datta et al, 1989]. L'ADNc spécifiant la désoxycytidine kinase humaine a été cloné et séquencé [Chottiner et al, 1991, numéro d'accession dans GENBANK : M60527], révélant des similarités entre DCK et les thymidine kinases des virus herpétiques [Harrison et al, 1991]. De même, la structure tridimensionnelle de la thymidine kinase de l'herpès a été résolue par radiocristallographie [Brown et al, 1995]. Le brevet américain US 6,063,376 décrit une seconde désoxycytidine kinase humaine appelée DCK2 qui possède 60 % d'identité avec DCK1.

Dans le procédé selon l'invention, on a mis à profit la propriété de mutateur conditionnel de DCK en présence d'analogues de nucléosides promutagènes pour soumettre son propre gène dck à un épisode de mutagénèse in vivo.

Ainsi, des bactéries de génotype *Δdeo tdk p*::*dckH*+ furent exposées soit à la 2-amino-2'-désoxyribosyl-purine (disoA) soit à la 2-amino-désoxyribosyl-2-hydroxy-purine (disoG), puis incubées sur milieu solide riche en présence de triméthoprime et de thymidine. Des colonies sont apparues suite à l'administration des deux composés à une fréquence de l'ordre de 10⁻⁸. Aucune colonie ne survint en absence de nucléoside promutagène.

Le séquençage des gènes de 7 plasmides mutants obtenus indépendamment (4 après mutagénèse par disoG, 3 par disoA) a mis en évidence deux mutations ponctuelles D133E et R104Q, chacune conférant l'acquisition de l'activité thymidine kinase par DCK. En outre, un plasmide réunissant les deux mutations dans un même allèle a été construit et introduit dans la souche β7117 de génotype *Δdeo tdk.* Il a permis la complémentation de la tdk inactivée par mutation ou délétion, exprimant donc aussi une activité thymidine kinase.

### Description

Ainsi, de manière générale, la présente invention se rapporte à un procédé d'évolution *in vivo* artificielle de protéines, ledit procédé permettant de faire évoluer *in vivo* une protéine X par complémentation d'une protéine apparentée Y, X et Y appartenant tous deux à la même classe de la nomenclature enzymatique EC ou à des classes voisines.

Un tel procédé permet de faire évoluer une protéine X de sorte à en modifier ses caractéristiques et comprend les étapes suivantes :
a) obtention de cellules comportant un génotype [protéine Y∗ : :protéine X+], par transformation de cellules [protéine Y∗ ] avec un acide nucléique comprenant le gène codant pour la protéine X, Y∗ signifiant que le gène codant pour Y a été inactivé, Y étant une protéine appartenant à une classe voisine de X possédant une activité voisine, les classes de X et Y se caractérisant en ce qu'elles possèdent au moins les trois premiers chiffres appartenant aux classes EC de la nomenclature internationale à 4 chiffres, lesdites cellules présentant un phénotype auxotrophe nécessitant pour survivre l'apport du produit de la réaction de Y sur son substrat dans le milieu de culture ;
a) exposer les cellules obtenues à l'étape a) à un mutagène,
b) mettre en culture lesdites cellules dans un milieu comprenant le substrat de Y, le produit de la réaction de Y sur son substrat étant nécessaire à la survie de ladite cellule,
c) sélectionner les cellules qui ont survécu à l'étape c) dans lesquelles la protéine X, modifiée par l'action dudit agent mutagène, complémente la déficience en la protéine Y.

A l'étape b), toute technique visant à augmenter la sensibilité de la cellule vis-à-vis d'un mutagène ou d'un promutagène, par exemple par expression d'une kinase, d'une phosphorylase ou d'une ADN pol exo-, peut être utilisée. De préférence, on utilise un vecteur d'expression de la DCK1 comportant les mutations D133E et R104Q décrite ci-après.

La présente invention a plus particulièrement pour objet un procédé pour faire évoluer une protéine X, sélectionnée parmi les kinases appartenant aux classes EC 2.7,1,-, les nucléotidyl transférases appartenant aux classes EC 2.7.7.-, et les phosphorylases appartenant aux classes EC 2.4.2.-, de sorte à obtenir une protéine modifiée présentant une activité élargie par rapport à la protéine X initiale, caractérisé en ce qu'il comprend :
a) l'obtention de cellules comportant un génotype [protéine Y∗ :: protéine X+], par transformation de cellules [protéine Y*] avec un acide nucléique comprenant le gène codant pour la protéine X, Y∗ signifiant que le gène codant pour Y a été inactivé, Y étant une protéine appartenant à une classe voisine de X possédant une activité voisine, les classes de X et Y se caractérisant en ce qu'elles possèdent au moins les trois premiers chiffres appartenant aux classes EC de la nomenclature internationale à 4 chiffres, lesdites cellules présentant un phénotype auxotrophe nécessitant pour survivre l'apport du produit de la réaction de Y sur son substrat dans le milieu de culture ;
b) exposer les cellules obtenues à l'étape a) à un analogue de nucléosides promutagènes,
c) mettre en culture lesdites cellules dans un milieu comprenant le substrat de Y, le produit de la réaction de Y sur son substrat étant nécessaire à la survie de ladite cellule,
d) sélectionner la ou les cellules qui ont survécu à l'étape c) dans lesquelles la protéine X, modifiée par l'action dudit analogue de nucléosides promutagènes, complémente la déficience de ladite cellule en la protéine Y.

Dans le cadre de l'invention, le terme «Y∗ » signifie que le gène codant pour Y a été inactivé, c'est à dire qu'il a été délété en tout ou en partie, ou inactivé par insertion d'une séquence ou par introduction d'une mutation. Il faut signaler que l'invention peut également être exécutée dans le cas de modification du gène Y conduisant à un phénotype du type Ts (température sensible). Dans ce cas, les cellules sont cultivées à des températures non permissibles pendant la phase de sélection (étapes c) et d)).

Parmi, les protéines que l'on cherche à faire évoluer, on peut citer notamment :
- les protéines appartenant à la famille des kinases, telles que par exemple

| Numéro EC | Nom selon la nomenclature internationale |
|---|---|
| 2.7.1,20 | Adenosine kinase. |
| 2.7,1.21 | Thymidine kinase. |
| 2.7.1.38 | Phosphorylase kinase. |
| 2.7.1.49 | Hydroxymethylpyrimidine kinase. |
| 2.7.1.74 | désoxycytidine kinase (DCK). |
| 2.7.4.6 | Nucleoside-diphosphate kinase. |
| 2.7.4.7 | Phosphomethylpyrimidine kinase. |
| 2.7.4.8 | Guanylate kinase. |
| 2.7.4.9 | Thymidylate kinase. |
| 2.7.4.10 | Nucleoside-triphosphate--adenylate kinase. |
| 2.7.4.11 | (Deoxy)adenylate kinase. |
| 2.7.4.12 | T2-induced deoxynucleotide kinase. |
| 2.7.4.13 | (Deoxy)nucleoside-phosphate kinase. |

- les nucléotidyl transférase, telles que par exemple

| **Numéro EC** | **Nom selon la nomenclature internationale** |
|---|---|
| 2.7.7.6 | DNA-directed RNA polymerase. |
| 2.7.7.7 | DNA-directed DNA polymerase. |
| 2.7.7.8 | Polyribonucleotide nucleotidyltransferase. |
| 2.7.7.19 | Polynucleotide adenylyltransferase. |
| 2.7.7.25 | tRNA adenylyltransferase. |
| 2.7.7.48 | RNA-directed RNA polymerase. |
| 2.7.7.49 | RNA-directed DNA polymerase. |
| 2.7.7.50 | mRNA guanylyltransferase. |

- les phosphorylases, telles que par exemple

| **Numéro EC** | **Nom selon la nomenclature internationale** |
|---|---|
| 2.4.2.1 | Purine-nucleoside phosphorylase. |
| 2.4.2.2 | Pyrimidine-nucleoside phosphorylase. |
| 2.4.2.3 | Uridine phosphorylase. |
| 2.4.2.4 | Thymidine phosphorylase. |
| 2.4.2.7 | Adenine phosphoribosyltransferase. |
| 2.4.2.8 | Hypoxanthine phosphoribosyltransferase. |
| 2.4.2.9 | Uracil phosphoribosyltransferase. |
| 2.4.2.15 | Guanosine phosphorylase. |
| 2.4.2.23 | Deoxyuridine phosphorylase. |
| 2.4.2.28 | 5'-methylthioadenosine phosphorylase. |

Bien entendu, d'autres enzymes, notamment les enzymes du métabolisme ou du catabolisme, peuvent faire l'objet d'une évolution grâce au procédé selon l'invention. Ces enzymes et leur numéro EC respectif sont répertoriés par le Committee of the International Union of Biochemistry and Molecular Biology (IIJBMB) à l'adresse suivante : http://expasy.proteome.org.au/enzyme/

Dans un mode de réalisation préféré, l'invention se rapporte à un procédé tel que défini ci-dessus dans lequel la protéine X a la propriété d'introduire des mutations dans l'ADN. La propriété de mutateur conditionnel de la protéine X en présence d'analogues de nucléosides promutagènes permet de soumettre son propre gène à un épisode de mutagénèse *in vivo.*

En ce sens, le procédé selon l'invention permet de faire évoluer une kinase X de sorte à en modifier ses caractéristiques, ledit procédé comprenant les étapes suivantes :
a) obtention de cellules comportant un génotype [kinaseY∗ ::kinaseX+], par transformation d'une cellule [kinaseY∗] avec un acide nucléique comprenant le gène codant pour la kinase X, Y∗ signifiant que le gène codant pour Y a été inactivé, Y étant une kinase appartenant à une classe voisine de X montrant une activité voisine, les classes de X et Y se caractérisant en ce qu'elles possèdent au moins les trois premiers chiffres appartenant aux classes EC 2.7.1.- de la nomenclature internationale à 4 chiffres, lesdites cellules présentant un phénotype auxotrophe nécessitant pour survivre l'apport du produit de la réaction de Y sur son substrat dans le milieu de culture ;
b) exposer les cellules obtenues à l'étape a) à un analogue de nucléosides promutagènes pendant un temps donné, la kinase X étant capable de phosphoryler ledit analogue,
c) mettre en culture lesdites cellules dans un milieu comprenant le substrat de Y, le produit de la réaction dudit substrat avec Y étant nécessaire à la survie de ladite cellule,
d) sélectionner les cellules qui ont survécu à l'étape c) dans lesquelles la kinase X, modifiée par l'action dudit analogue de nucléoside promutagène, complémente la déficience en la kinase Y.

Par « complément », on entend la suppression du phénotype auxotrophe résultant de l'inactivation du gène Y.

Lesdites cellules sont des cellules procaryotes ou eucaryotes, de préférence *E. coli.* Dans le cas où la protéine X est une kinase, le substrat est sélectionné parmi les nucléosides et leurs analogues.

Avantageusement, la kinase X est une déoxycytidine kinase appartenant à la classe EC 2.7.1.74. La kinase Y est de préférence une kinase n'appartenant pas à EC 2.7.1.74, notamment une thymidine kinase (TDK) appartenant à la classe EC 2.7.1.21. Dans la mesure où X est une phosphorylase ou une polymérase, Y est une phosphorylase ou une polymérase différente de X.
Ainsi, le procédé évoqué ci-dessus peut comprendre les étapes suivantes :
a) obtention d'une bactérie *E. coli* Δ*deo tdk p::dckH*+*,*
   mise en culture des cellules obtenues à l'étape a) dans un milieu comprenant :
   - un agent mutagène sélectionné parmi les analogues de nucléosides promutagènes et du triméthoprime qui bloque la synthèse du thymidylate par la thymidylate synthase ;
   - et la thymidine qui est nécessaire à la survie de ladite cellule,
b) sélection des cellules qui ont survécu à l'étape b) dans lesquelles la DCKH, modifiée par l'action dudit analogue promutagène, complémente la déficience en TDK.

Avantageusement, la kinase X est une désoxycytidine kinase, notamment la DCK1 humaine de séquence déposée dans GENBANK sous le numéro d'accession M60527 comportant au moins une mutation sélectionnée parmi les mutations D133E et R104Q. La séquence double mutante de DCK1 (SEQ ID No1) est :

| | |
|---|---|
| 1 | MATPPKRSCP SFSASSEGTR IKKISIEGNI AAGKSTFVNI LKQLCEDWEV VPEPVARWCN |
| 61 | VQSTQDEFEE LTMSQKNGGN VLQMMYEKPE RWSFTFQTYA CLSRIRAQLA SLNGKLKDAE |
| 121 | KPVLFFERSV YSDRYIFASN LYESECMNET EWTIYQDWHD WMNNQFGQSL ELDGIIYLQA |
| 181 | TPETCLHRIY LRGRNEEQGI PLEYLEKLHY KHESWLLHRT LKTNFDYLQE VPILTLDVNE |
| 241 | DFKDKYESLV EKVKEFLSTL |

En outre, la kinase X est de préférence capable d'activer un analogue de nucléoside promutagène, lequel analogue introduit des mutations dans son propre gène. A l'issu du procédé, la kinase X mutée est capable de remplacer (complémentation) la kinase Y et présente donc une activité élargie par rapport à son activité initiale.

Ainsi, dans un deuxième aspect, l'invention vise une protéine X mutée, notamment une kinase X mutée, susceptible d'être obtenue à partir du procédé décrit ci-dessus, caractérisée en ce qu'elle présente une activité élargie par rapport à la protéine X initiale (ou à la kinase X initiale). Il peut s'agir par exemple de la kinase DCK1 humaine mentionnée ci-dessus comportant au moins une mutation sélectionnée parmi les mutations D133E et R104Q. L'invention porte également sur un acide nucléique comprenant une séquence codant pour la kinase DCK1 humaine telle que définie ci-dessus et un vecteur comprenant cette séquence codante, ladite séquence pouvant être fusionnée à un promoteur efficace dans les cellules eucaryotes et/ou procaryotes.

De préférence, le vecteur est un plasmide qui peut être introduit dans une bactérie telle que par exemple *E. coli* par transformation; le vecteur se maintient dans la bactérie de manière stable ou transitoire.
L'invention porte également sur une cellule hôte comprenant un vecteur exposé ci-dessus.

Un autre aspect de l'invention concerne l'utilisation d'une kinase, explicitée précédemment, dans un procédé tel que défini ci-dessus pour rendre actif un analogue de nucléoside promutagène. Plus généralement, l'invention vise l'utilisation d'une kinase mentionnée ci-dessus dans tout procédé d'hypermutagénèse, pour convertir des nucléosides, naturellement réfractaires à la phosphorylation enzymatique, en leur dérivé 5' phosphate respectif.
L'invention porte également sur l'utilisation concomitante d'une kinase selon l'une des revendications 12 et 13 et d'autres enzymes tels que AMK et/ou la transdesoxyribosylase (NTD) pour élargir in *vivo* la gamme de la mutagénèse à l'aide de promutagènes.

En outre, il faut signaler que le vecteur mentionné ci-dessus peut être utilisé pour la préparation d'un médicament destiné à la thérapie génique pour permettre l'incorporation d'analogues de nucléosides dans l'ADN.

L'invention vise également un procédé de mutagénèse *in vivo* d'une séquence d'ADN déterminée, ladite séquence d'ADN étant dans une cellule, comprenant les étapes consistant à :
- effectuer la mutation par insertion d'au moins un type de nucléosides promutagènes dans ladite séquence, la cellule exprimant au moins un système enzymatique permettant l'insertion dudit nucléotide promutagène dans l'ADN,
- et détecter la présence de la séquence mutée,
caractérisé en ce que le système enzymatique comprend une kinase telle que définie ci-dessus.

Ce procédé permet de faire évoluer une protéine spécifique de la cellule. En ce sens, on inactive un gène codant pour une protéine apparentée à ladite protéine spécifique, la protéine apparentée étant nécessaire à la survie de la cellule, et on détecte la complémentation après mutation du gène codant pour ladite protéine spécifique. Ladite protéine spécifique et ladite protéine apparentée sont de préférence des enzymes appartenant à une classe voisine ayant en commun au moins les trois premiers chiffres de la nomenclature internationale EC à 4 chiffres.
Ces protéines sont sélectionnées parmi les kinases appartenant aux classes EC 2.7.1. : on peut également envisager les nucléotidyl transférases appartenant aux classes EC 2.7.7. -notamment les polymérases, et les phosphorylases appartenant aux classes EC 2.4.2.- en utilisant des cribles appropriés. Avantageusement, lesdites protéines ont la propriété de pouvoir faire évoluer leur propre gène.

L'invention porte également sur la souche d'*E. coli* β 7151 de génotype *Δdeo tdk* comprenant un vecteur exprimant la DCK mutée D133E déposée le 21 février 2001 à la CNCM (Collection Nationale de Cultures de Microorganismes, 25, rue du Docteur Roux, 75724 Paris cedex 15, France) sous le numéro d'accession I-2631.
L'invention porte également sur une souche d'*E*. *coli* β 7134 de génotype *Δdeo tdk* comprenant un vecteur exprimant la DCK humaine déposée le 21 février 2001 à la CNCM sous le numéro d'accession I-2630.
L'invention porte également sur une souche d'*E. coli* β7338 de génotype *Δdeo tdk* comprenant un vecteur exprimant la DCK double mutante D133E et R104Q déposée le 27 mars 2001 à la CNCM sous le numéro d'accession I-2650.

### Légendes

Figure 1: crible permettant la sélection d'une activité thymidine kinase chez *Escherichia coli.*

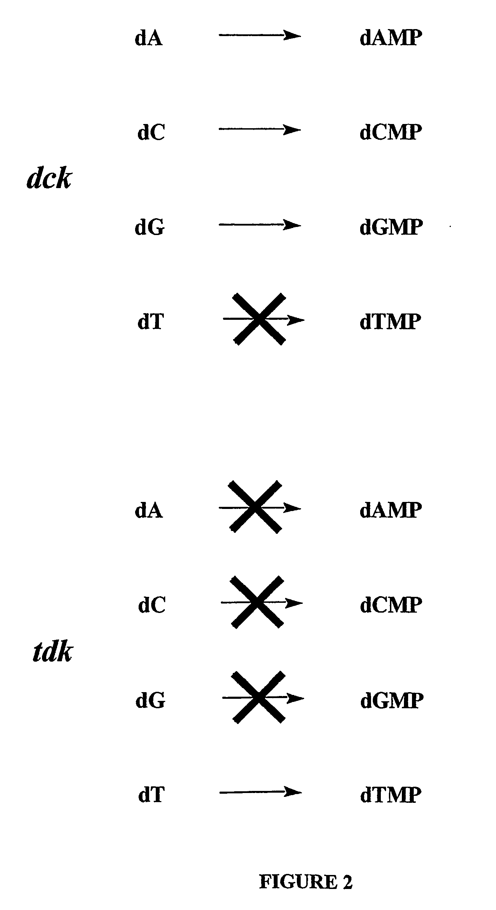

Figure 2: spécificités comparées de la thymidine kinase d'*Escherichia coli* et de la désoxycytidine kinase humaine.

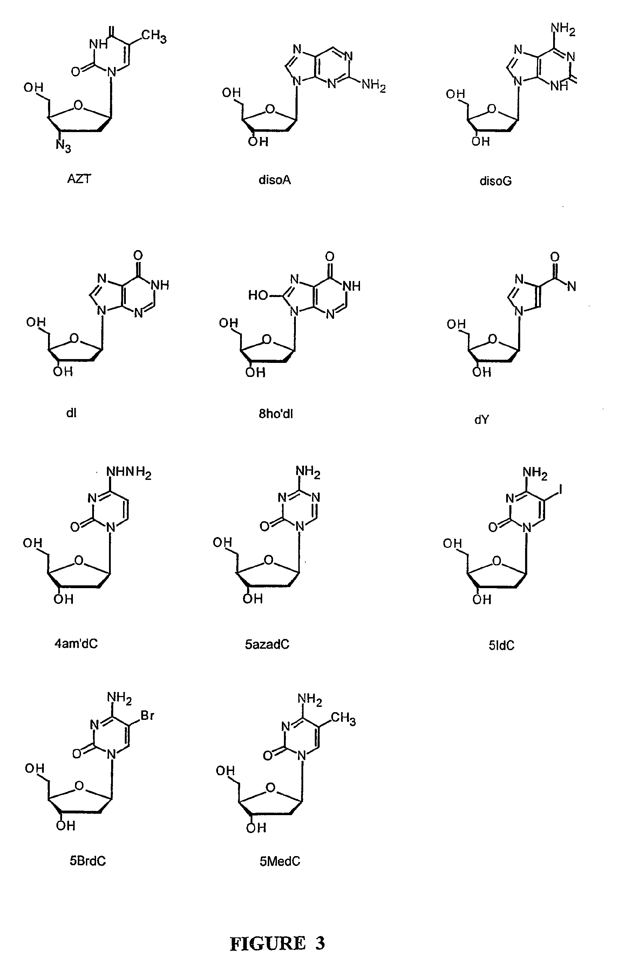

Figure 3: exemples d'analogues de nucléosides

### Matériels et méthodes

**Composés chimiques :** les composés 3'-azido-3'-désoxythymidine (AZT), 2'-désoxyinosine (dI), N4-amino-2'-désoxycytidine (désoxyribonucléoside de la 2-hydroxy-4-hydrazino-pyrimidine, désigné 4am'dC), 5-aza-2'-désoxycytidine (5azadC), 5-iodo-2'-désoxycytidine (5IdC), 5-bromo-2'-désoxycytidine (5BrdC) and 5-methyl-2'-désoxycytidine (5MedC) ont été achetés chez Sigma. La 2-désoxy-iso-adénosine (disoA) (2-amino-9-(2'-désoxy-B-D-ribofuranosyl)purine) a été préparée par transglycosylation enzymatique en utilisant un extrait brut de N-deoxyribosyltransferases de *Lactobacillus leichmannii.* La synthèse de la 2'-désoxyisoguanosine (2-hydroxy-6-amino-9-(2'-deoxy-β-D-ribofuranosyl)purine) a été réalisée par fermeture du cycle du 5-amino-1-(2'-désoxy-13-D-ribofuranosyl)imidazole-4-carboxamide. La préparation du 1-(2'-désoxyribofuramosyl)-imidazole-4-carboxamide (dY) a été décrite dans Pochet et al, 1995.

**Culture des souches bactériennes :** les bactéries ont été cultivées dans un milieu riche Luria-Bertani (LB) ou dans un milieu minimum supplémenté avec 2g/l de glucose (MS). Les mêmes milieux de croissance ont été solidifiés avec 15g/l d'agar (Difco) pour la préparation des boîtes de Pétri. Les cultures liquides et solides ont été incubées à 37°C. Dans certains cas, des antibiotiques ont été rajoutés aux concentrations suivantes : 100mg/l de carbenicilline, 25mg/l de kanamycine, 15mg/l de tétracycline. Le triméthoprime a été utilisé à une concentration de 100 mg/l dans un milieu LB supplémenté avec 0,3 mM de thymidine. Pour l'induction de l'expression du gène, l'isopropyl-β-D-thiogalactoside (IPTG) a été rajouté à 0,5 mM.

**Souches et constructions plasmidiques :** la liste des souches *E. coli* K12 utilisées et les plasmides construits dans le cadre de la présente invention est donnée dans le tableau 1 ci-après.

**Tableau 1 : souches bactériennes et plasmides**

| Souche | Phénotype | Construction |
|---|---|---|
| MG1655 | F⁻λ⁻ | B. Bachmann |
| KU8 | *trxB*::*Tn*10Kan *ΔserB* zjj::*Tn*10 | Uhland et al. |
| SØ928 | *Δdeo Δlac thi upp udp ton* | P. Nygaard |
| SØ5110 | *cdd*::*Tn*10 | P. Nygaard |
| CC101 | *ara Δ*(*lac proB)13* | Cupples and Miller |
| | F' *lacZ:*Glu461*am proB*⁺ | |
| CC102 | *ara Δ(lac proB)13* | Cupples and Miller |
| | F' *lacZ:*Glu461Gly *proB*⁺ | |
| CC103 | *ara Δ(lac proB)13* | Cupples and Miller |
| | F' *lacZ:*Glu461Gln *proB*⁺ | |
| CC104 | *ara Δ(lac proB)13* - | Cupples and Miller |
| | F'*lacZ:*Glu461Ala *proB*⁺ | |
| CC105 | *ara Δ(lac proB)13* | Cupples and Miller |
| | F' *lacZ:*Glu461Val *proB*⁺ | |
| CC106 | *ara Δ*(*lac proB)13* | Cupples and Miller |
| | F'*lacZ:*Glu461Lys *proB⁺* | |
| β7069 | *tdk* | Mutant MG1655 (résistance spontanée à l'AZT) |
| β7117 | *Δdeo tdk* | Transductions séquentielles de β7069 avec des lysats P1 de KU8 et SØ928 |
| β 7134 | *Δdeo tdk* | Transformation de β7117 |
| | pDCK1(*bla⁺ lacIQ dck⁺*) | |
| β7151 | *Δdeo tdk* | Plasmide pDCK D133E |
| β7320 | *Δdeo tdk cdd*::*Tn*10 | Transduction de β7117 avec le lysat P1 de SØ5110 |
| β7334 | *Δdeo tdk cdd::Tn*10 | Transformation de β7320 |
| | pSUTrc (*kan*⁺ *lacI^{Q}*) | |
| β7335 | *Δdeo tdk cdd::Tn*10 | Transformation de β7320 |
| | pSUDCK1 (*kan*⁺ *lacI^{Q} dck*⁺) | |
| β7336 | *Δdeo tdk cdd::Tn*10 | Transformation de β7320 |
| | pSUDCK2 (*kan*⁺ *IacI^{Q} dck:*D133E) | |
| β7337 | *Δdeo tdk cdd::Tn*10 | Transformation de β7320 |
| | pSUDCK3 (*kan*⁺ *lacI^{Q} dck:*R104Q) | |
| β7338 | *Δdeo tdk cdd::Tn*10 | Transformation de β7320 |
| | pSUDCK4 (*kan*⁺ *lacI^{Q} dck*:D133E-R104Q) | |
| β7339 | *Δdeo tdk cdd*::*Tn*10 | Double transformation de β7320 |
| | pSUTrc (*kan*⁺ *lacI^{Q}*) | |
| | pAK1 (*bla*⁺ *lacI^{Q} adk*⁺) | |
| β7340 | *Δdeo tdk cdd*::*Tn*10 | Double transformation de β7320 |
| | pSUDCK1 (*kan+ lacIQ dck*⁺) | |
| | pAK1 (*bla⁺ lacIQ adk⁺*) | |
| β7341 | *Δdeo tdk cdd*::*Tn*10 | Transformation de β7336 |
| | pSUDCK2 (*kan*⁺ *IacI^{Q} dck:*D133E) | |
| | pAK1 (*bla*⁺ *lacI^{Q} adk*⁺) | |
| β7342 | *Δdeo tdk edd::Tn*10 | Double transformation de β7320 |
| | pSUDCK3 (*kan*⁺ *lacI^{Q} dck:*R104Q) | |
| | pAK1 (*bla*⁺ *lacI^{Q} adk*⁺) | |
| β87343 | *Δdeo tdk cdd::Tn*10 | Double transformation de β7320 |
| | pSUDCK4 (*kan*⁺ *lacI^{Q} dck:*D133E-R104Q) | |
| | pAK1 (*bla*⁺ *IacI^{Q} adk*⁺) | |
| β87344 | *Δdeo tdk cdd*::*Tn*10 | Double transformation de β7320 |
| | pSUTrc (*kan*⁺ *lacI^{Q}*) | |
| | pAKT39A (*bla*⁺ *lacI^{Q} adk:*T39A) | |
| β7345 | *Δdeo tdk cdd*::*Tn*10 | Double transformation de β7320 |
| | pSUDCKl (*kan*⁺ *lacI^{Q} dck*⁺) | |
| | pAKT39A *(bla*⁺ *lacl^{Q} adk:*T39A) | |
| β7346 | *Δdeo tdk cdd::Tn*10 | Double transformation de β7320 |
| | pSUDCK2 (*karn*⁺ *lacI^{Q} dck:*D133E) | |
| | pAKT39A (*bla*⁺ *lacI^{Q} adk:* T39A) | |
| β7347 | *Δdeo tdk cdd*::*Tn*10 | Double transformation de β7320 |
| | pSUDCK3 (*kan*⁺ *lacI^{Q} dck:*R104Q) | |
| | pAKT39A (*bla*⁺ *lacI^{Q} adk:*T39A) | |
| β7348 | *Δdeo tdk cdd*::*Tn*10 | Double transformation de β7320 |
| | pSUDCK4 (*kan*⁺ *lacI^{Q} dck:*D133E-R104Q) | |
| | pAKT39A (*bla*⁺ *lacI^{Q} adk:*T39A) | |
| β7351 | *ara Δ*(*lac proB)13* | Transformation de CC101 |
| | F' *lacZ:*Glu461 *am proB*⁺ | |
| | pSUDCK2 (*kan*⁺ *lacI^{Q} dck:*D133E) | |
| β7352 | *ara* Δ(*lac proB*)*13* | Transformation de CC102 |
| | F' *lacZ:*Glu461Gly *proB*⁺ | |
| | pSUDCK2 (*kan*⁺ *lacI^{Q} dck:*D133E) | |
| β7353 | *ara Δ*(*lac proB)13* | Transformation de CC103 |
| | F'*lacZ:*Glu461Gln *proB*⁺ | |
| | pSUDCK2 (*kan*⁺ *lacI^{Q} dck:*D133E) | |
| β7354 | *ara* Δ(*lac proB*)*13* | Transformation de CC104 |
| | F' *lacZ:*Glu461Ala *proB*⁺ | |
| | pSUDCK2 (*kan*⁺ *lacI^{Q} dck:*D133E) | |
| β7355 | *ara Δ*(*lac proB)13* | Transformation de CC105 |
| | F' *lacZ:*Glu461Val *proB*⁺ | |
| | pSUDCK2 (*kan*⁺ *lacI^{Q} dck:*D133E) | |
| β7356 | *ara* Δ(*lac proB)13* | Transformation de CC106 |
| | F' *lacZ:*Glu461Lys *proB*⁺ | |
| | pSUDCK2 (*kan*⁺ *lacI^{Q} dck:*D133E) | |
| β7357 | *ara* Δ(*lac proB)13* | Double transformation de CC101 |
| | F' *lacZ:*Glu461*am proB*⁺ | |
| | pSUDCK2 (*kan*⁺ *lacI^{Q} dck:*D133E) | |
| | pAK1 (*bla*⁺ *lacI^{Q} adk*⁺) | |
| β7358 | *ara* Δ(*lac proB)13* | Double transformation de CC102 |
| | F' *lacZ:*Glu461Gly *proB*⁺ | |
| | pSUDCK2 (*kan*⁺ *lacI^{Q} dck:*D133E) | |
| | pAK1 (*bla*⁺ *lacI^{Q} adk*⁺) | |
| β7359 | *ara Δ*(*lac proB)13* | Double transformation de CC103 |
| | F' *lacZ:*Glu461Gln*proB*⁺ | |
| | pSUDCK2 (*kan*⁺ *lacI^{Q} dck:*D133E) | |
| | pAK1 (*bla*⁺ *lacI^{Q} adk*⁺) | |
| β7360 | *ara* Δ(*lac proB)13* | Double transformation de CC104 |
| | F' *lacZ:*Glu461Ala *proB*⁺ | |
| | pSUDCK2 (*kan*⁺ *lacI^{Q} dck:*D133E) | |
| | pAK1 (*bla*⁺ *lacI^{Q} adk*⁺) | |
| β7361 | *ara* Δ(*lac proB)13* | Double transformation de CC105 |
| | F' *lacZ:*Glu461Val *proB*⁺ | |
| | pSUDCK2 (*kan*⁺ *lacI^{Q} dck:*D133E) | |
| | pAK1 (*bla*⁺ *lacI^{Q} adk*⁺) | |
| β7362 | *ara* Δ(*lac proB)13* | Double transformation de CC106 |
| | F'*lacZ:*Glu461Lys *proB*⁺ | |
| | pSUDCK2 (*kan*⁺ *lacl^{Q} dck:*D133E) | |
| | pAK1 (*bla*⁺ *lacI^{Q} adk*⁺) | |

| Plasmides | | |
|---|---|---|
| pTrc99A | *bla*⁺ *lacI^{Q}* | ColE1 réplicon (Pharmacia) |
| pDCK1 | *bla*⁺ *lacI^{Q} dck*⁺ | Bouzon & Marlière |
| pDCK2 | *bla*⁺ *lacI^{Q} dck:*D133E | Mutagénèse *In vivo* / disoG de β7134 |
| pDCK3 | *bla*⁺ *lacI^{Q} dck:*R104Q | Mutagénèse *In vivo* / disoA de β7134 |
| pDCK4 | *bla*⁺ *lacI^{Q} dck:*D133E-R104Q | Substitution d'un fragment *Sac*I-*BamH*I de 466 bases de pDCK3 par celui de pDCK2 |
| pSUTrc | *kan*⁺ *lacI^{Q}* | Clonage du fragment *Sph*I-*BamH*I de pTrc99A dans pSU39 |
| pSUDCK1 | *kan*⁺ *lacI^{Q} dck*⁺ | Clonage du fragment *Sph*I-*BamH*I de pDCK1 dans pSU39 |
| pSUDCK2 | *kan*⁺ *lacI^{Q} dck:*D133E | Clonage du fragment *Sph*I-*BamH*I de pDCK2 dans pSU39 |
| pSUDCK3 | *kan*⁺ *lacI^{Q} dck:*R104Q | Clonage du fragment *Sph*I-*BamH*I de pDCK3 dans pSU39 |
| pSUDCK4 | *kan*⁺ *lacI^{Q} dck:*D133E-R104Q | Clonage du fragment *Sph*I-*BamH*I de pDCK4 dans pSU39 |
| pAK1 | *bla*⁺ *lacI^{Q} adk*⁺ | Dr T. Okajima |
| pAKT39A | *bla*⁺ *lacI^{Q} adk:*T39A | Dr. T. Okajima |

La souche β7069 a été sélectionnée comme étant un mutant spontané résistant à l'AZT de MG1655 cultivée sur boîte LB supplémentée avec 30 µM d'AZT. Le phénotype *tdk* a été déterminé par l'absence de croissance sur milieu riche Mueller-Hinton (MH) comprenant du triméthoprime et de la thymidine. On a contrôlé que la mutation β7069 tdk ne réverse pas spontanément en repiquant les cellules sur le milieu MH supplémenté avec du triméthoprime et de la thymidine après 20 générations en LB. La souche β117 *Δdeo tdk* a été obtenue après deux transductions P1 consécutives. Les bactéries β7069 ont d'abord été infectées avec le lysat P1 provenant de KU8 dans le but de transférer la délétion *ΔserB* et le marqueur proximal *Tn10.* Les clones résistants à la tétracycline ont été sélectionnés et testés pour leur auxotrophie à la sérine. Un de ces clones a ensuite été infecté avec le lysat P1 provenant de SØ928 et les prototrophes à la sérine ont été sélectionnés sur milieu minimum. Tous les transductants Ser⁺ sélectionnés comportent la délétion de l'opéron *deo* car ils sont incapables de croître en milieu minimum avec de la thymidine comme seule source de carbone. Le plasmide pDCK4 a été construit en substituant le fragment SacI-BamHI de 466 bases de long de pDCK3 par celui de pDCK2. La présence des mutations D133E et R104Q sur pDCK4 a été déterminée par séquençage.

**Sélection *in vivo* des mutants DCK :** une culture de 12 heures des cellules β7134 en milieu MS supplémenté avec 50 mg/l de carbenicilline a été diluée 100 fois dans le même milieu et cultivée en aération jusqu'au moment où une turbidité de 0,100 (600nm) soit atteinte. La culture a ensuite été diluée 25 fois dans le même milieu avec ou sans IPTG et supplémentée avec des concentrations variables (5 à 30µM) des analogues de nucléosides promutanégiques disoG et disoA puis cultivée en aération pendant 18 heures. Les cellules ont été rincées puis étalées sur des boîtes MH supplémentées avec du triméthoprime, de la thymidine et de l'IPTG. Des colonies sont apparues après 36 heures d'incubation à 37°C. Aucune colonie n'a été obtenue en l'absence d'analogue de nucléoside.

**Test de détermination de la concentration minimale inhibitrice (CMI)** : la CMI des analogues de nucléosides a été déterminée conformément aux méthodes classiques avec les modifications suivantes : une culture bactérienne de 12 heures en milieu MS avec les antibiotiques appropriés a été diluée 100 fois dans le même milieu et cultivée à 37°C en aération jusqu'à l'obtention d'une turbidité d'environ 0,1 OD. (600nm). La culture a ensuite été diluée 100 fois dans le même milieu supplémenté avec IPTG et distribuée sur microplaque 96 puits dans un volume final de 100 µl dans une série de dilutions de 2 en 2 d'analogues de nucléotide. Chaque analogue a été testé deux fois. Après une incubation pendant 18 heures à 37°C avec agitation, le CMI a été déterminé comme correspondant à la concentration la plus faible en analogue pour laquelle aucune turbidité n'est détectable.

**Test de réversion :** Des cellules des souches lac CC101 à CC106 ont été transformées, soit avec le plasmide pSUDCK2 seul, soit avec les plasmides pSUDCK2 et pAK1. Les transformations ont été cultivées pendant 12 heures en milieu minimum comprenant 0,2% de glucose avec les antibiotiques appropriés pour la maintenance des plasmides. La culture a ensuite été diluée 100 fois dans le même milieu et cultivée jusqu'à l'apparition d'une DO de 0,1 (600nm). Ensuite, les cultures ont été diluées 100 fois dans le même milieu supplémenté avec 0,5 mM IPTG puis diluées 10 fois avec une solution 10 fois concentrée de l'analogue de nucléoside à tester. Les cellules ont été cultivées pendant 18 heures à 37°C avant la mise en culture sur boîte. Le dénombrement des cellules viables a été réalisé sur milieu solide LB. Les révertants Lac+ ont été sélectionnés en milieu minimum contenant 0,2% de lactose comme source de carbone. La fréquence de mutation a été définie comme étant le ratio : nombre de cellules mutantes sur nombre de cellules viables. Diverses concentrations finales des analogues nucléosidiques correspondant aux CMI/2, CMI/4, CMI/10 et CMI/20 ont été testées, car la sensibilité des souches dérivées de celles de Miller vis-à-vis des différents analogues n'est pas identique à celle des souches dérivées de MG1655. La fréquence de mutations a été déterminée en utilisant les cultures obtenues avec la concentration la plus élevée en analogue nucléosidique permettant une croissance visible après 18 heures.

### Exemple 1 : crible sélectif

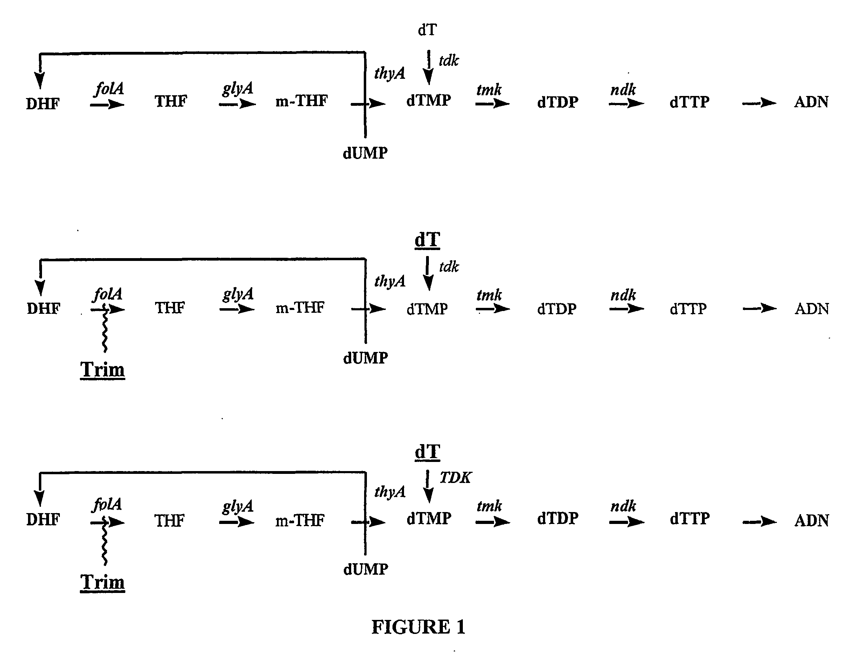

*Escherichia coli* ne possède pas d'activité désoxynucléoside kinase à l'exception d'une thymidine kinase, codée par le gène tdk, très spécifique de la thymidine et de la désoxyuridine. Nous avions montré précédemment comment l'introduction de l'activité désoxycytidine kinase chez *E. coli* ouvre une voie métabolique inexistante dans cet organisme, et permet aux désoxynucléosides naturels et à des analogues structuraux d'accéder au pool des monomères de l'ADN [Bouzon & Marlière, 1997]. Une souche portant un allèle défectif du gène tdk ne peut utiliser la thymidine comme source de thymidylate (dTMP) et sa croissance dépend de l'intégrité de la voie de synthèse *de novo* de celui-ci (Figure 1). La synthèse de dTMP à partir de dUMP est catalysée par la thymidylate synthase (gène thyA). Elle peut être bloquée par le triméthoprime qui par inhibition de l'activité de la dihydrofolate réductase conduit à l'épuisement du pool intracellulaire du 5,10-méthylène-tétrahydrofolate, donneur du groupement méthyle dans la réaction. Nous avons d'abord montré qu'une souche dont le gène tdk est inactivé ne prolifère pas sur milieu riche en présence de triméthoprime et de thymidine. Ce crible sélectif qui impose le maintien d'une activité thymidine kinase a été mis en oeuvre pour faire évoluer l'activité de la désoxycytidine kinase humaine chez *E. coli.* On le trouve schématisé dans la Figure 1.

### Exemple 2 : sélection de mutants dckH présentant une activité élargie

Nous avons mis à profit la propriété de mutateur conditionnel de DCK en présence d'analogues de nucléosides promutagènes pour soumettre son propre gène dck à un épisode de mutagénèse *in vivo.* Ainsi, des bactéries de génotype *Δdeo tdk p::dckH*+ furent exposées soit à la 2'-désoxy-iso-adénosine (disoA) soit à la 2'-désoxy-iso-guanosine (disoG), puis incubées sur milieu solide riche en présence de triméthoprime et de thymidine. Des colonies sont apparues suite à l'administration des deux composés à une fréquence de l'ordre de 10⁻⁸. Aucune colonie ne survint en absence de nucléoside promutagène.

Le séquençage des gènes de 7 plasmides mutants obtenus indépendamment (4 après mutagénèse par disoG, 3 par disoA) mit en évidence deux mutations ponctuelles D133E et R104Q, chacune conférant l'acquisition de l'activité thymidine kinase par DCK. Ces allèles sont désignés dckH*. Un plasmide réunissant les deux mutations dans un même allèle, dckH**, fut construit et introduit dans la souche β7117 de génotype Δ*deo tdk.* Il permit la complémentation de la mutation tdk, exprimant donc aussi une activité thymidine kinase. Les différents allèles sélectionnés sont répertoriés dans le Tableau 2 ci-après.

**Tableau 2: mutations du gène hétérologue dckH supprimant le phénotype tdk de Escherichia coli.**

| Mutagène | Mutation détectée | Fréquence | Site de la mutation | substitution acide aminé |
|---|---|---|---|---|
| disoG | C → A | 4/4 | codon 133 GAC | Asp -> Glu |
| | | | | |
| disoA | C→ A | 1/3 | codon 133 GAC | Asp -> Glu |
| | C → G | 1/3 | codon 133 GAC | Asp → Glu |
| | G → A | 1/3 | codon 104 CGA | Arg -> Gln |

Les détails de la sélection et de l'analyse génétique sont donnés dans la section Matériels et Méthodes.

### Exemple 3 : propriétés fonctionnelles des mutants dckH

La toxicité d'analogues de nucléosides, déviant soit par le sucre soit par la base, a été évaluée dans des souches de génotype *Δdeo tdk cdd* exprimant sur un plasmide les différents allèles de dckH, allèle sauvage, D133E, R104Q et double mutant.

Le marqueur Δdeo correspond à l'inactivation de l'opéron catabolique des désoxynucléosides et le marqueur cdd à l'inactivation de la désoxycytidine désaminase; ces marqueurs évitent que les analogues de nucléosides apportés dans le milieu engendrent des dérivés autres que le dérivé phosphorylé souhaité par action de DCK; ils permettent l'emploi de doses plus faibles des analogues. Les résultats sont indiqués dans le Tableau 3 ci-après.

**Tableau 3: toxicité d'analogues de deoxynucléoside induite par la deoxycytidine kinase sur Escherichia coli.**

| Analogue de nucléoside | Souche et allèle dck *Δdeo tdk cdd::Tn*10 | | | | |
|---|---|---|---|---|---|
| | β7334 | β7335 | β7336 | β7337 | β7338 |
| | none | wt | D133E | R104Q | D133E |
| | | | | | R104Q |
| ddA | 80 | 80 | 80 | 80 | 80 |
| ddU | > | > | > | > | > |
| ddT | > | > | > | > | > |
| ddC | > | > | > | > | > |
| ddI | 80 | 40 | 40 | 20 | 40 |
| araC | > | 160 | 20 | 20 | 320 |
| AZT | > | > | 640 | 640 | 1280 |
| 5'-amino-dT | > | > | > | > | > |
| disoA | > | > | 10 | > | > |
| disoG | > | > | > | > | > |
| dI | > | > | 1.25 | > | 320 |
| disoI | > | > | > | > | > |
| 8ho'dI(*) | > | > | 1280 | > | > |
| DAP | > | > | > | > | > |
| d-oxanosine | > | > | 80 | > | > |
| dY | > | > | > | > | > |
| dJ | > | > | > | > | > |
| amino-dC | > | > | 20 | 2.5 | 20 |
| 5-aza-dC | > | 1.25 | 1.25 | 1.25 | 1.25 |
| 5-iodo-dC | > | > | > | > | > |
| 5-bromo-dC | > | > | > | > | > |
| 5-methyl-dC | > | > | > | > | > |
| 5-methyl-disoC | > | > | > | > | > |
| 5-chloro-dU | > | > | > | > | > |
| 5-bromo-dU | > | > | > | > | > |
| 5-iodo-dU | > | > | > | > | > |
| 5-hm-dU | > | > | > | > | > |

| | | | | | |
|---|---|---|---|---|---|
| (*) désoxyribonucléoside de la 8-hydroxy-hypoxanthine | | | | | |

Les concentrations inhibitrices minimales, exprimées en microM, ont été déterminées comme indiqué dans la section Matériels et Méthodes.
Chaque dosage a été effectué trois fois : aucune toxicité n'a pu être détectée à plus haute concentration en analogue, 1,28 mM.
Le génotype détaillé des souches hôtes de chaque allèle est indiqué au Tableau 1.

Bien que conduisant toutes deux à l'acceptation de la thymidine comme substrat, les mutations ponctuelles D133E et R104Q ont des effets contrastés sur la phosphorylation des différents analogues testés.

Les souches sauvages d'*E. coli* sont sensibles à de faibles concentrations d'AZT, tandis que les souches tdk, qui ont perdu l'activité thymidine kinase, y sont réfractaires [Elwell et al, 1987]. Les tests rapportés dans le Tableau 3 indiquent que l'AZT n'est pas substrat de DCK wt, mais que le mutant D133E active l'analogue de telle façon qu'on détecte une toxicité à haute concentration de l'analogue (CMI= 1280 microM). Il est probable que cette toxicité provient de l'incorporation de l'AZT triphosphate par l'ADN polymérase et le blocage de l'élongation par ce terminateur de chaine après les actions successives de la dTMP kinase et de la nucléoside diphosphokinase sur l'AZT monophosphate.

Poursuivant l'analyse des résultats du Tableau 3, la mutation D133E entraîne une forte toxicité de disoA, (CMI=10 microM). La mutation R104Q n'a pas d'effet vis-à-vis de ce composé. De même, la mutation D133E entraîne une très forte toxicité de la désoxyinosine, dI (CMI= 1 microM). Le désoxyribonucléoside de la 2-hydroxy-4-hydrazino-pyrimidine (désignée 4am'dC) apparait meilleur substrat du mutant R104Q que du mutant D133E, les deux mutations causant une très forte augmentation de la toxicité de l'analogue. La 5-aza-désoxycytidine (désignée 5azadC) est toxique à très faible concentration (CMI< 1.25 microM) quel que soit l'allèle de DCK.

Globalement, l'allèle dckH-D133E se montre le plus intéressant, augmentant la sensibilité pour le plus grand nombre d'analogues. La combinaison des deux mutations D133E et R104Q mène à un spectre d'activité apparemment intermédiaire entre chacun des deux mutants individuels.

### Exemple 4 : diversification métabolique par coexpression de gènes hétérologues

L'absence d'effet toxique par un analogue de nucléoside vis-à-vis d'une souche d'*E. coli* exprimant le gène dckH ou l'un de ses allèles mutants peut avoir plusieurs raisons, si on suppose que tout effet toxique résulte de l'incorporation de monomères erronés dans les chaînes d'ADN : (i) l'analogue n'est pas substrat ou est un mauvais substrat de l'enzyme DCK; (ii) l'analogue est phosphorylé en monophosphate par DCK mais les étapes ultérieures de phosphorylation en diphosphate puis en triphosphate échouent; (iii) l'analogue triphosphate n'est pas substrat de l'ADN polymérase.

Il est connu que les nucléoside monophosphate kinases, qui produisent les diphosphates à partir des triphosphates acceptent le ribose et le désoxyribose, mais sont très spécifiques de la base. On s'attendait donc à ce que la coproduction d'un enzyme formant une variété élargie de monophosphates (allèles mutés DCK) et d'un enzyme formant une variété élargie de diphosphates dans la même cellule d'*E. coli* révèle les substrats nucléosidiques portant des bases déviantes susceptibles d'être phosphorylés en monophosphates par DCK wt ou DCK muté mais dont la conversion en diphosphate ne puisse être catalysée par les enzymes d'*E*. *coli.*

L'adénosine monophosphate kinase des eucaryotes (AMK) présente une parenté de structure avec les UMP/CMP kinases des bactéries [Okajima et al, 1993]. Sa fonction physiologique serait de catalyser l'échange de phosphate entre AMP et ATP:

AMP + ATP <--> ADP + ADP ;

l'enzyme agit également sur un substrat portant le désoxyribose:

dAMP + ATP <--> dADP + ADP.

Le mutant T39A du gène de l'enzyme du poulet (amkG*) a été construit par mutagénèse dirigée en se guidant sur des comparaisons de séquence, par un groupe au Japon [Okajima et al, 1993]. In vitro, l'activité de AMK sur CMP est inférieure à 1 % de son activité sur AMP. La mutation T39A modifie le spectre d'activité de l'enzyme et augmente significativement la conversion de CMP et UMP [Okajima et al, 1993].

Nous avons exprimé conjointement dans le contexte génétique *Δdeo tdk cdd* chacun des quatre allèles de dckH avec chacun des deux allèles wt et T39A de amkG, et testé la toxicité des différents analogues de nucléosides déjà testés précédemment (Tableau 4 ci-après).

**Tableau 4: toxicité d'analogues de deoxynucléoside induite par la coexpression de la deoxycytidine kinase and de l'adenosine monophosphate kinase dans Escherichia coli.**

| Analogue de Nucléoside | Strain dckH allele amkG allele | | | | | | | | | |
|---|---|---|---|---|---|---|---|---|---|---|
| | β7339 | β7340 | β7341 | β7342 | β7343 | β7344 | β7345 | β7346 | β7347 | β7348 |
| | none | wt | D133 | R104 | D133E | none | wt | D133 | R104 | D133E |
| | | | E | Q | R104Q | | | E | Q | R104Q |
| | wt | wt | wt | wt | wt | T39A | T39A | T39A | T39A | T39A |
| AZT | > | > | > | > | > | > | > | 1280 | > | > |
| disoA | > | > | 5 | > | 1280 | > | > | 10 | > | > |
| disoG | > | > | > | > | > | > | > | > | > | > |
| dI | > | > | ≤1.25 | > | 1280 | > | > | ≤1.25 | > | 1280 |
| 8oxodI | > | > | 1280 | > | > | > | > | 1280 | > | > |
| dY | > | > | > | > | > | > | > | 1280 | > | > |
| 4am'dC | > | > | 80 | ≤1.25 | 80 | > | > | 640 | 5 | 320 |
| 5azadC | > | ≤1.25 | ≤1.25 | ≤1.25 | ≤1.25 | > | ≤1.25 | ≤1.25 | ≤1.25 | ≤1.25 |
| 5IdC | > | > | ≤1.25 | ≤1.25 | ≤1.25 | > | ≤1.25 | ≤1.25 | ≤1.25 | ≤1.25 |
| 5BrdC | > | ≤1.25 | ≤1.25 | ≤1.25 | ≤1.25 | > | ≤1.25 | ≤1.25 | ≤1.25 | ≤1.25 |
| 5MedC | > | ≤1.25 | ≤1.25 | ≤1.25 | 5 | > | ≤1.25 | ≤1.25 | ≤1.25 | ≤1.25 |

Les concentrations inhibitrices minimum, exprimées en microM, ont été déterminées comme indiqué dans la section Matériels and méthodes.

Chaque expérience a été effectuée trois fois : aucune toxicité n'a pu être détectée à plus haute concentration en analogue, 1280 microM.
Le génotype détaillé des souches hôtes de chaque allèle est indiqué au Tableau 3.

Il est apparu que la coexpression des deux gènes eucaryotes entraîne la conversion métabolique des dérivés 5-halogénés (5BrdC, 5IdC) et 5-méthylé (5MedC) de la désoxycytidine dC en dérivés inhibiteurs pour les bactéries recombinantes, tandis que l'expression d'un seul des deux gènes laisse les bactéries réfractaires aux mêmes analogues. La très grande toxicité de l'analogue lorsqu'il y a expression concomitante de DCK et AMK indique que DCK phosphoryle ces substrats mais que c'est l'étape ultérieure de phosphorylation par les monophosphates kinases qui est limitante.

Comme on le voit dans le Tableau 4, la conjonction de l'allèle DCK-D133E et de l'allèle AMK-T39A entraîne une toxicité des souches d"*E*. *coli* qui les portent vis-à-vis du nucléoside simplifié dY, désoxyribosyl-imidazole-carboxamide [Pochet et al, 1995]. Les effets mutagènes de la base Y avaient été démontrés ex vivo au cours de réactions d'amplification PCR, causant en particulier des transitions A:T->G:C et des transversions A:T->T:A [Sala et al, 1996]. La toxicité de dY à 1 mM vis-à-vis des souches rapportées ici s'accompagne d'une augmentation du même spectre de mutations *in vivo.*

### REFERENCES

Bouzon M. & Marliere P. (1997) Human deoxycytidine kinase as a conditional mutator in Escherichia coli. Comptes Rendus de 1 Academie des Sciences - Serie III, Sciences de la Vie. 320(6):427-34

Brown DG. Visse R. Sandhu G. Davies A. Rizkallah PJ. Melitz C. Summers WC. Sanderson MR. (1995) Crystal structures of the thymidine kinase from herpes simplex virus type-1 in complex with deoxythymidine and ganciclovir. Nature Structural Biology. 2(10):876-81

Cazaux C, Tiraby M, Loubiere, Haren L, Klatzmann D & Tiraby G (1998) Phosphorylation and cytotoxicity of therapeutic nucleoside analogues: a comparison of alpha and gamma herpesvirus thymidine kinase suicide genes. Cancer Gene Therapy 5(2):83-91

Chottiner EG. Shewach DS. Datta NS. Ashcraft E. Gribbin D. Ginsburg D. Fox IH. Mitchell BS. (1991) Cloning and expression of human deocytidine kinase cDNA Proceedings of the National Academy of Sciences of the United States of America. 88(4):1531-5

Datta NS. Shewach DS. Hurley MC. Mitchell BS. Fox IH. (1989) Human T-lymphoblast deoxycytidine kinase: purification and properties. Biochemistry. 28(1):114-23

Elwell LP et al (1987) Antibacterial activity and mechanism of action of 3'-azido-3'-deoxythymidine (BW A509U). Antimicrobial Agents & Chemotherapy 31(2):274-80

Harrison PT. Thompson R. Davison AJ. (1991) Evolution of herpesvirus thymidine kinases from cellular deoxycytidine kinase. Journal of General Virology. 72:2583-6

Johansson M, Van Rompay AR, Degrève B, Balzarini J & Karlsson A (1999) Cloning and characterization of the multisubstrate deoxyribonucleoside kinase of Drosophila melanogaster. J Biol Chem 274:23814-23819

Mullen CA (1994) Metabolic suicide genes in gene therapy. [Review] Pharmacology & Therapeutics 63(2):199-207

Okajima T. Tanizawa K. Fukui T. (1993) Site-directed mutagenesis of AMP-binding residues in adenylate kinase. FEBS Letters. 334(1):86-8

Pochet S., Dugué L., Meier A. & Marlière P. (1995) "Enzymatic synthesis of 1-(2-deoxy-β-D-ribofuranosyl)-imidazole-4-carboxamide, a simplified DNA building block" Bioorganic & Medicinal Chemistry Letters 5, 1679-1684.

Sala M., Pezo V., Pochet S. & Wain-Hobson S. (1996) "Ambiguous base pairing of the purine analogue 1-(2-deoxy-β-D-ribofuranosyl)-imidazole-4-carboxamide during PCR" Nucleic Acids Research 24, 3302-3306.

## Revendications

1. Procédé pour faire évoluer une protéine X, sélectionnée parmi les kinases appartenant aux classes EC 2.7.1.-, les nucléotidyl transférases appartenant aux classes EC 2.7.7.-, et les phosphorylases appartenant aux classes EC 2.4.2.-,
de sorte à obtenir une protéine modifiée présentant une activité élargie par rapport à la protéine X initiale, **caractérisé en ce qu'**il comprend :
a) l'obtention de cellules comportant un génotype [protéine Y∗ :: protéine X+], par transformation de cellules [protéine Y*] avec un acide nucléique comprenant le gène codant pour la protéine X, Y∗ signifiant que le gène codant pour Y a été inactivé, Y étant une protéine appartenant à une classe voisine de X possédant une activité voisine, les classes de X et Y **se caractérisant en ce qu'**elles possèdent au moins les trois premiers chiffres appartenant aux classes EC de la nomenclature internationale à 4 chiffres, lesdites cellules présentant un phénotype auxotrophe nécessitant pour survivre l'apport du produit de la réaction de Y sur son substrat dans le milieu de culture ;
b) exposer les cellules obtenues à l'étape a) à un analogue de nucléosides promutagènes,
c) mettre en culture lesdites cellules dans un milieu comprenant le substrat de Y, le produit de la réaction de Y sur son substrat étant nécessaire à la survie de ladite cellule,
d) sélectionner la ou les cellules qui ont survécu à l'étape c) dans lesquelles la protéine X, modifiée par l'action dudit analogue de nucléosides promutagènes, complémente la déficience de ladite cellule en la protéine Y.

2. Procédé selon la revendication 1 **caractérisé en que** en ce que la protéine X est capable d'activer un analogue de nucléosides promutagènes, lequel analogue introduit des mutations dans son propre gène.

3. Procédé selon l'une des revendications 1 à 2 pour faire évoluer une kinase X de sorte à obtenir une kinase modifiée présentant une activité élargie par rapport à la kinase X initiale, **caractérisé en ce qu'**il comprend :
a) l'obtention de cellules comportant un génotype [kinase Y∗ :: kinaseX+], par transformation d'une cellule [kinase Y*] avec un acide nucléique comprenant le gène codant pour la kinase X, Y∗ signifiant que le gène codant pour Y a été inactivé, Y étant une kinase appartenant à une classe voisine de X montrant une activité voisine, les classes de X et Y **se caractérisant en ce qu'**elles possèdent au moins les trois premiers chiffres appartenant aux classes EC 2.7.1.- de la nomenclature internationale à 4 chiffres, lesdites cellules présentant un phénotype auxotrophe nécessitant pour survivre l'apport du produit de la réaction de Y sur son substrat dans le milieu de Culture ;
b) exposer les cellules obtenues à l'étape a) à un analogue de nucléosides promutagènes pendant un temps donné, la kinase X étant capable de phosphoryler ledit analogue,
c) mettre en culture lesdites cellules dans un milieu comprenant le substrat de Y, le produit de la réaction de Y sur son substrat étant nécessaire à la survie de ladite cellule,
d) sélectionner les cellules qui ont survécu à l'étape c) dans lesquelles la kinase X, modifiée par l'action dudit analogue de nucléosides promutagènes, complémente la déficience en la kinase Y.

4. Procédé selon l'une des revendications 1 à 3 **caractérisé en ce que** lesdites cellules sont des cellules procaryotes ou eucaryotes, de préférence *E. coli.*

5. Procédé selon l'une des revendications 1 à 4 **caractérisé en ce que** ledit substrat est sélectionné parmi les nucléosides et leurs analogues.

6. Procédé selon l'une des revendications 3 à 5 **caractérisé en ce que** la kinase X est une déoxycytidine kinase (DCK) appartenant à la classe EC 2.7.1.74.

7. Procédé selon l'une des revendications 3 à 6 **caractérisé en ce que** la kinase Y est une thymidine kinase (TDK) appartenant à la classe EC 2.7.1.21.

8. Procédé selon l'une des revendications 1 à 4 **caractérisé en ce qu'**il comprend les étapes suivantes :
a) l'obtention d'une bactérie *E. coli* Δ*deo tdk p::dckH*+*,*
mise en culture des cellules obtenues à l'étape a) dans un milieu comprenant :
- un agent mutagène sélectionné parmi les analogues de nucléosides promutagènes et du triméthoprime qui bloque la synthèse du thymidylate par la thymidylate synthase ;
- et de la thymidine qui est nécessaire à la survie de ladite cellule,
b) la sélection des cellules qui ont survécu à l'étape b) dans lesquelles la DCKH, modifiée par l'action dudit analogue de nucléosides promutagènes, complémente la déficience en TDK.

9. Procédé selon l'une des revendications 3 à 8 **caractérisé en ce que** la kinase X est une désoxycytidine kinase, de préférence la DCK1 humaine possédant la séquence déposée dans GENBANK sous le numéro d'accession M60527 comportant au moins une mutation sélectionnée parmi les mutations D133E et R104Q.

10. Kinase X appartenant à la classe EC 2.7.1.74. mutée susceptible d'être obtenue selon le procédé de l'une quelconque des revendications 1 à 9, **caractérisée en ce qu'**elle a acquis l'activité d'une thymidine kinase (TDK) appartenant à la classe EC 2.7.1.21.

11. Kinase X mutée, **caractérisée en ce qu'**elle possède la séquence déposée dans GENBANK sous le numéro d'accession M60527 et **en ce qu'**elle comporte au moins une mutation sélectionnée parmi les mutations D133E et R104Q.

12. Acide nucléique comprenant la séquence codant pour la kinase selon la revendication 11.

13. Vecteur d'expression comprenant la séquence codant pour la kinase selon la revendication 11.

14. Vecteur selon la revendication 13 **caractérisé en ce que** ladite séquence est fusionnée à un promoteur efficace dans les cellules eucaryotes et/ou procaryotes.

15. Vecteur selon l'une des revendications 13 et 14 **caractérisé en ce qu'**il s'agit d'un plasmide capable de transformer et de se maintenir chez *E. coli.*

16. Cellule hôte comprenant un vecteur selon l'une des revendications 13 à 15.

17. Utilisation d'une kinase obtenue selon l'une quelconque des revendications 10 ou 11, pour l'activation d'un analogue de nucléosides promutagènes,

18. Utilisation d'une kinase obtenue selon l'une des revendications 10 ou 11, dans un procédé d'hypermutagénèse.

19. Utilisation d'une kinase obtenue selon l'une des revendications 10 et 11, pour convertir des nucléosides, naturellement réfractaires à la phosphorylation enzymatique, en leur dérivé 5' phosphate respectif.

20. Utilisation concomitante d'une kinase selon l'une des revendications 10 et 11, avec d'autres enzymes tels que l'adénosine monophosphate kinase (AMK) et/ou la transdesoxyribosylase (NTD) pour élargir in *vivo* la gamme de la mutagénèse à l'aide de promutagènes.

21. Utilisation d'un vecteur selon l'une des revendications 13 à 15, pour la préparation d'un médicament destiné à la thérapie génique pour permettre l'incorporation d'analogues de nucléosides dans l'ADN.

22. Procédé de mutagénèse d'une séquence d'ADN déterminée, ladite séquence d'ADN étant dans une cellule isolée, comprenant les étapes consistant à :
- effectuer la mutation par insertion d'au moins un type de nucléosides promutagènes dans ladite séquence, la cellule exprimant au moins un système enzymatique permettant l'insertion dudit nucléoside promutagène dans l'ADN,
- et détecter la présence de la séquence mutée,
**caractérisé en ce que** le système enzymatique comprend une kinase selon l'une des revendications 10 ou 11.

23. Utilisation selon la revendication 20 pour faire évoluer une protéine spécifique, ladite protéine étant exprimé de la cellule, **caractérisé en ce qu'**on inactive un gène codant pour une protéine apparentée à ladite protéine spécifique, la protéine apparentée étant nécessaire à la survie de la cellule, et on détecte la complémentation après mutation du gène codant pour ladite protéine spécifique.

24. Utilisation selon la revendication 23 **caractérisé en que** ladite protéine spécifique et ladite protéine apparentée sont des enzymes appartenant à une classe voisine ayant en commun au moins les trois premiers chiffres de la nomenclature internationale EC à 4 chiffres.

25. Utilisation selon la revendication 24 **caractérisé en que** lesdites protéines ont la propriété de pouvoir faire évoluer leur propre gène.

26. Souche d'*E. coli* β 7151 de génotype Δ*deo tdk* comprenant un vecteur exprimant la DCK mutée D133E déposée à la CNCM sous le numéro d'accession I-2631.

27. Souche d'*E. coli* β 7134 de génotype Δ*deo tdk* comprenant un vecteur exprimant la DCK humaine déposée à la CNCM sous le numéro d'accession I-2630.

28. Souche d'*E. coli* β7338 de génotype Δ*deo tdk* comprenant un vecteur exprimant la DCK double mutante D133E et R104Q déposée à la CNCM sous le numéro d'accession I-2650.

## Claims

1. Process for the evolution of a protein X selected from the kinases belonging to the EC 2.7.1.- classes, the nucleotidyl transferases belonging to the EC 2.7.7.- classes and the phosphorylases belonging to the EC 2.4.2.- classes,
in order to obtain a modified protein having an extended activity compared with the initial protein X, **characterized in that** it comprises:
a) obtaining cells comprising a genotype [protein Y*::protein X+] by transformation of cells [protein Y-] with a nucleic acid comprising the gene coding for the protein X, Y* signifying that the gene coding for Y has been inactivated, Y being a protein belonging to a class related to X, having a related activity, the classes of X and Y being **characterized in that** they possess at least the first three figures belonging to the EC classes of 4-figure international nomenclature, said cells having an auxotrophic phenotype requiring for survival the addition of the product of the reaction of Y on its substrate in the culture medium;
b) exposing the cells obtained in stage a) to a promutagenic nucleoside analogue,
c) culture of said cells in a medium comprising the substrate of Y, the product of the reaction of Y on its substrate being necessary for the survival of said cell,
d) selecting the cell or cells which have survived stage c), in which the protein X, modified by the action of said promutagenic nucleoside analogue, complements the deficiency in the protein Y.

2. Process according to claim 1 **characterized in that** the protein X is capable of activating a promutagenic nucleoside analogue, which analogue introduces mutations into its own gene.

3. Process according to one of claims 1 to 2 for the evolution of a kinase X in order to obtain a modified kinase having an extended activity compared with the initial kinase X,
**characterized in that** it comprises:
a) obtaining cells comprising a genotype [kinase Y*::kinase X+] by transformation of a cell [kinase Y*] with a nucleic acid comprising the gene coding for the kinase X, Y* signifying that the gene coding for Y has been inactivated, Y being a kinase belonging to a class related to X, showing a related activity, the classes of X and Y being **characterized in that** they possess at least the first three figures belonging to EC classes 2.7.1.- of the 4-figure international nomenclature, said cells having an auxotrophic phenotype requiring for survival the addition of the product of the reaction of Y on its substrate in the culture medium;
b) exposure of the cells obtained in stage a) to a promutagenic nucleoside analogue over a given period of time, the kinase X being capable of phosphorylating said analogue,
c) culture of said cells in a medium comprising the substrate of Y, the product of the reaction of Y on its substrate being necessary for the survival of said cell,
d) selecting the cells which have survived stage c), in which the kinase X, modified by the action of said promutagenic nucleoside analogue, complements the kinase Y deficiency.

4. Process according to one of claims 1 to 3 **characterized in that** said cells are prokaryotic or eukaryotic cells, preferably *E coli.*

5. Process according to one of claims 1 to 4 **characterized in that** said substrate is selected from the nucleosides and their analogues.

6. Process according to one of claims 3 to 5 **characterized in that** the kinase X is a deoxycitidine kinase (DCK) belonging to EC class 2.7.1.74.

7. Process according to one of claims 3 to 6 **characterized in that** the kinase Y is a thymidine kinase (TDK) belonging to EC class 2.7.1.21.

8. Process according to one of claims 1 to 4 **characterized in that** it comprises the following stages:
a) obtaining an *E. coli* Δ*deo tdk p::dckH*+ bacterium,
culturing of the cells obtained in stage a) in a medium comprising:
- a mutagenic agent selected from the promutagenic nucleoside and trimethoprim analogues which block thymidylate synthesis by thymidylate synthase;
- and thymidine which is necessary for the survival of said cell,
b) selecting the cells which have survived in stage b) in which DCKH, modified by the action of said promutagenic nucleoside analogue, complements TDK deficiency.

9. Process according to one of claims 3 to 8 **characterized in that** the kinase X is a deoxycytidine kinase, preferably human DCK1 possessing the sequence filed in GENBANK under accession number M60527 comprising at least one mutation selected from the mutations D133E and R104Q.

10. Mutated kinase X belonging to EC class 2.7.1.74 capable of being obtained according to the process according to any one of claims 1 to 9 **characterized in that** it has acquired the activity of a thymidine kinase (TDK) belonging to EC class 2.7.1.21.

11. Mutated kinase X **characterized in that** it has the sequence filed in GENBANK under accession number M60527 and **in that** it comprises at least one mutation selected from the mutations D133E and R104Q.

12. Nucleic acid comprising the sequence coding for the kinase according to claim 11.

13. Expression vector comprising the sequence coding for the kinase according to claim 11.

14. Vector according to claim 13 **characterized in that** said sequence is fused to an effective promoter in eukaryotic and/or prokaryotic cells.

15. Vector according to one of claims 13 and 14 **characterized in that** it is a plasmid capable of transforming and being maintained in *E. coli.*

16. Host cell comprising a vector according to one of claims 13 to 15.

17. Use of a kinase obtained according to any one of claims 10 and 11 for the activation of a promutagenic nucleoside analogue.

18. Use of a kinase obtained according to one of claims 10 or 11 in a hypermutagenesis process.

19. Use of a kinase obtained according to one of claims 10 and 11 in order to convert nucleosides, which are naturally refractory to enzymatic phosphorylation, to their respective 5' phosphate derivative.

20. Concomitant use of a kinase according to one of claims 10 and 11 with other enzymes such as adenosine monophosphate kinase (AMK) and/or transdeoxyribosylase (NTD) in order to extend *in vivo* the range of mutagenesis by means of promutagens.

21. Use of a vector according to one of claims 13 to 15 for preparing a medicament intended for gene therapy in order to allow the incorporation of nucleoside analogues into DNA.

22. Mutagenesis process of a specific DNA sequence, said DNA sequence being in an isolated cell, comprising stages consisting of:
- carrying out the mutation by insertion of at least one type of promutagenic nucleosides into said sequence, the cell expressing at least one enzymatic system allowing the insertion of said promutagenic nucleotide into the DNA,
- and detecting the presence of the mutated sequence,
**characterized in that** the enzymatic system comprises a kinase according to one of claims 10 and 11.

23. Use according to claim 20 for the evolution of a specific protein, said protein being expressed by the cell, **characterized in that** a gene coding for a protein related to said specific protein is inactivated, the related protein being necessary for the survival of the cell, and the complementation is detected after mutation of the gene coding for said specific protein.

24. Use according to claim 23 **characterized in that** said specific protein and said related protein are enzymes belonging to a related class sharing at least the first three figures of the 4-figure EC international nomenclature.

25. Use according to claim 24 **characterized in that** said proteins have the property of being able to cause their own gene to evolve.

26. *E. coli* strain β 7151 of genotype *Δdeo tdk* comprising a vector expressing the D133E-mutated DCK deposited at the CNCM under accession number I-2631.

27. *E. coli* strain P 7134 of genotype *Δdeo tdk* comprising a vector expressing human DCK deposited at the CNCM under accession number I-2630.

28. *E. coli* strain β 7338 of genotype *Δdeo tdk* comprising a vector expressing the D133E and R104Q double mutant DCK deposited at the CNCM under accession number I-2650.

## Patentansprüche

1. Verfahren zur Weiterentwicklung eines Proteins X, ausgewählt aus den Kinasen, welche zu den Klassen EC 2.7.1.- gehören, den Nukleotidyltransferasen, welche zu den Klassen EC 2.7.7.- gehören, und den Phosphorylasen, welche zu den Klassen EC 2.4.2.-gehören, um ein modifiziertes Protein zu erhalten, welches eine erhöhte Aktivität im Vergleich zu dem ursprünglichen Protein X aufweist, **dadurch gekennzeichnet, dass** es umfasst:
a) die Herstellung von Zellen mit einem Genotyp [Protein Y*:: Protein X+] durch Transformation der Zellen [Protein Y*] mit einer Nukleinsäure, welche das Gen, das für das Protein X kodiert, enthält, wobei Y* bedeutet, dass das für Y kodierende Gen inaktiviert wurde, wobei Y ein Protein ist, die zu einer verwandten Klasse von X mit vergleichbarer Aktivität gehört, die Klassen den X und Y sind **dadurch gekennzeichnet, dass** sie mindestens die ersten 3 Ziffern der EC-Klassen der vierstelligen internationalen Nomenklatur gemeinsam haben, besagten Zellen weisen einen auxotrophen Phenotyp auf, der die Zugabe des Produktes der Reaktion von Y mit seinem Substrat im Kulturmedium zum Überleben benötigt,
b) das Exponieren der Zellen aus Schritt a) mit einem promutagenen Nukleosidanalogon,
c) das Kultivieren besagter Zellen in einem Medium, welches das Substrat von Y enthält, wobei das Produkt der Reaktion von Y mit seinem Substrat, für das Überleben besagter Zelle notwendig ist,
d) das Selektieren von der oder den Zelle(n), welche den Schritt c) überlebt haben, wobei das Protein X, welches durch die Behandlung mit besagtem promutagenen Nukleosidanalogon modifiziert wurde, das Fehlen des Proteins Y in der besagten Zelle komplementiert.

2. Verfahren gemäß Anspruch 1, **dadurch gekennzeichnet, dass** das Protein X in der Lage ist ein promutagenes Nukleosidanalogons zu aktivieren, welches Mutationen in seinem eigenen Gen einführt.

3. Verfahren gemäß Ansprüche 1 oder 2 zur Weiterentwicklung einer Kinase X um eine modifizierte Kinase mit einer erweiterten Aktivität im Vergleich zu der ursprünglichen Kinase X zu erhalten, **dadurch gekennzeichnet, dass** es umfasst:
a) die Herstellung von Zellen mit einem Genotyp [KinaseY*:: KinaseX+] durch Transformation der Zellen [KinaseY*] mit einer Nukleinsäure, welche das Gen, das für die Kinase X kodiert, enthält, wobei Y* bedeutet, dass das für Y kodierende Gen inaktiviert wurde, wobei Y eine Kinase ist, die zu einer verwandten Klasse von X mit vergleichbarer Aktivität gehört, die Klassen den X und Y sind **dadurch gekennzeichnet, dass** sie mindestens die ersten 3 Ziffern der Klassen EC 2.7.1.- der vierstelligen internationalen Nomenklatur aufweisen, besagten Zellen weisen einen auxotrophen Phenotyp auf, der die Zugabe des Produktes der Reaktion von Y mit seinem Substrat in dem Kulturmedium zum Überleben benötigt,
b) das Exponieren der Zellen aus Schritt a) mit einem promutagenen Nukleosidanalogon für eine bestimmte Zeit, wobei die Kinase X in der Lage ist das besagte Analogon zu phosphorylieren,
c) das Kultivieren besagter Zellen in einem Medium, welches das Substrat von Y enthält, wobei das Produkt der Reaktion von Y mit seinem Substrat für das Überleben besagter Zelle notwendig ist,
d) das Selektieren der Zellen, welche den Schritt c) überlebt haben, in denen die Kinase X, welche durch die Behandlung mit besagtem promutagenen Nukleosidanalogon modifiziert wurde, das Fehlen der Kinase Y in besagter Zelle komplementiert.

4. Verfahren gemäß einem der Ansprüche 1 bis 3, **dadurch gekennzeichnet, dass** die besagte Zellen Prokaryoten oder Eukaryoten sind, vorzugsweise *E. coli.*

5. Verfahren gemäß einem der Ansprüche 1 bis 4, **dadurch gekennzeichnet, dass** das besagte Substrat aus den Nukleosiden und deren Analoga ausgewählt ist.

6. Verfahren gemäß einem der Ansprüche 3 bis 5, **dadurch gekennzeichnet, dass** die Kinase X eine Desoxycytidinkinase (DCK) ist, welche zu der Klasse EC 2.7.1.74 gehört.

7. Verfahren gemäß einem der Ansprüche 3 bis 6, **dadurch gekennzeichnet, dass** die Kinase Y eine Thymidinkinase (TDK) ist, welche zu der Klasse EC 2.7.1.21 gehört.

8. Verfahren gemäß einem der Ansprüche 1 bis 4, **dadurch gekennzeichnet, dass** es die folgenden Schritte umfasst:
a) das Erhalten einer Bakterie *E. Coli Δdeo tdk p::dckH*+*,*
das Kultivieren von den Zellen aus Schritt a) in einem Medium umfassend:
- ein mutagenes Agens, ausgewählt aus den promutagenen Nukleosid- und Trimethoprimanaloga, welche die Synthese des Thymidylat durch die Thymidylatsynthase blockiert;
- und Thymidin, welches für das Überleben besagter Zelle notwendig ist,
b) das Selektieren der Zellen, welche den Schritt b) überlebt haben, in denen die DCKH, die durch die Behandlung mit besagtem promutagenen Nukleosidanalogon modifiziert wurde, das Fehlen der TDK komplementiert.

9. Verfahren gemäß einem der Ansprüche 3 bis 8, **dadurch gekennzeichnet, dass** die Kinase X eine Desoxycytidinkinase ist, bevorzugt die menschliche DCK1, welche die Sequenz, die unter der Nummer M60527 in der GENBANK eingetragenen ist, aufweist und welche mindestens eine Mutation ausgewählt aus den Mutationen D133E und R104Q enthält.

10. Kinase X der Klasse 2.7.1.74, die gemäß des Verfahrens von einem der Ansprüche 1 bis 9 verändert wurde, **dadurch gekennzeichnet, dass** sie die Aktivität einer Thymidinkinase (TDK) erworben hat, welche zu der Klasse EC 2.7.1.21 gehört.

11. Mutierte Kinase X, **dadurch gekennzeichnet, dass** sie die Sequenz, die in der GENBANK unter der Nummer M60527 eingetragen ist, aufweist und dass, sie mindestens eine Mutation ausgewählt aus den Mutationen D133E und R104Q enthält.

12. Nukleinsäure, welche eine für die Kinase gemäß Anspruch 11 kodierende Sequenz enthält.

13. Expressionsvektor, welcher eine Sequenz enthält, die für die Kinase gemäß Anspruch 11 kodiert.

14. Vektor gemäß Anspruch 13, **dadurch gekennzeichnet, dass** die besagte Sequenz an einen Promoter fusioniert ist, der in eukaryotischen und/oder prokaryotischen Zellen wirksam ist.

15. Vektor gemäß einem der Ansprüche 13 und 14, **dadurch gekennzeichnet, dass** es um einem Plasmid geht das in der Lage ist *E.coli* zu transformieren und darin vermehrt zu werden.

16. Wirtszelle, welche ein Vektor gemäß einem der Ansprüche 13 bis 15 enthält.

17. Verwendung einer Kinase, welche gemäß einem der Ansprüche 10 oder 11 hergestellt wurde, zur Aktivierung eines promutagenen Nukleosidanalogons.

18. Verwendung einer Kinase, welche gemäß einem der Ansprüche 10 oder 11 hergestellt wurde, in einem Hypermutageneseverfahren.

19. Verwendung einer Kinase, welche gemäß einem der Ansprüche 10 und 11 hergestellt wurde, zum Umwandeln von Nukleosiden, welche naturgemäß nicht einer enzymatischen Phosphorylierung unterliegen, in deren entsprechende 5' Phosphatderivate.

20. Gleichzeitige Verwendung einer Kinase gemäß einem der Ansprüche 10 und 11, mit anderen Enzymen, wie Adenosin Monophosphat Kinase (AMK) und/oder Transdesoxyribosylase (NDT) zur Erweiterung der Möglichkeiten der Mutagenese mit Hilfe von Promutagenen *in vivo.*

21. Verwendung eines Vektors gemäß einem der Ansprüche 13 bis 15, zur Herstellung eines Medikaments mit Anwendung in der Gentherapie, das den Einbau von Nukleosidanaloga in die DNS erlaubt.

22. Mutageneseverfahren einer bestimmten DNS Sequenz, wobei besagte DNS Sequenz in einer einzelnen Zelle ist, wobei das Verfahren folgende Schritte umfasst:
- Durchführen der Mutation, durch den Einbau mindestens einer Art von promutagenen Nukleosiden in die besagte Sequenz, wobei die Zelle mindestens ein enzymatisches System exprimiert, das den Einbau des besagten promutagenen Nukleosids in die DNS erlaubt,
- und die Detektion der mutierten Sequenz,
**dadurch gekennzeichnet, dass** das enzymatische System eine Kinase gemäß einem der Ansprüche 10 oder 11 enthält.

23. Verwendung gemäß Anspruch 20 zur Entwicklung eines spezifischen Proteins, wobei besagtes Protein in einer Zelle exprimiert wird, **dadurch gekennzeichnet, dass** man ein Gen inaktiviert, welches für ein verwandtes Protein des besagten spezifischen Proteins kodiert, wobei das verwandte Protein für das Überleben der Zelle notwendig ist, und Komplementierung wird detektiert, nachdem das für das besagte spezifische Protein kodierenden Gen mutiert wurde.

24. Verwendung gemäß Anspruch 23, **dadurch gekennzeichnet, dass** das besagte spezifische Protein und das besagte verwandte Protein Enzyme sind, welche zu einer vergleichbaren Klasse gehören, die mindestens die drei ersten Ziffern der vierstelligen Internationale EC Nomenklatur gemeinsam haben.

25. Verwendung gemäß Anspruch 24, **dadurch gekennzeichnet, dass** das besagte Proteine die Eigenschaft aufweisen, dass sie ihre eigene Gene weiterentwickeln können.

26. Der Stamm *E*. *coli* β 7151 des Genotyps *Δdeo tdk,* der einen Vektor umfasst, welcher die D133E-mutierte DCK exprimiert, und in der CNCM unter der Nummer I-2631 eingetragen wurde.

27. Der Stamm *E. coli* β 7134 des Genotyps *Δdeo tdk*, der einen Vektor umfasst, welcher die menschliche DCK exprimiert, und in der CNCM unter der Nummer I-2630 eingetragen wurde.

28. Der Stamm *E. coli* β 7338 des Genotyps *Δdeo tdk,* der einen Vektor umfasst, welcher die DCK Doppelmutante D133E und R104Q exprimiert und in der CNCM unter der Nummer I-2650 eingetragen wurde.
